# EUROPÄISCHE PATENTSCHRIFT

(11) **EP 0 481 920 B1**
(45) Veröffentlichungstag und Bekanntmachung des Hinweises auf die Patenterteilung: **19.07.1995**
(21) Anmeldenummer: 91810786.3
(22) Anmeldetag: 09.10.1991
(51) Int. Cl.: C07F 9/655, C07F 9/6553, A61K 31/665, A61K 31/67

(54) **Neue Benzoheterocyclylalkylaminoalkandiphosphonsäuren**
Benzoheterocyclylalkylaminoalkane diphosphonic acids
Acides benzohétérocyclylalkylaminoalcane diphosphoniques

(30) Priorität: 18.10.1990 CH 3332/90
(43) Veröffentlichungstag der Anmeldung: 22.04.1992
(73) Patentinhaber: CIBA-GEIGY AG, 4002 Basel (CH)
(72) Erfinder: Fröstl, Wolfgang, Dr., CH-4056 Basel (CH); Jaeggi, Knut A., Dr., CH-4054 Basel (CH)

(56) Entgegenhaltungen:
- EP-A- 0 084 822
- EP-A- 0 085 321
- EP-A- 0 170 228
- EP-A- 0 337 706

## Beschreibung

Die Erfindung betrifft Benzoheterocyclylalkylaminoalkandiphosphonsäuren der Formel I 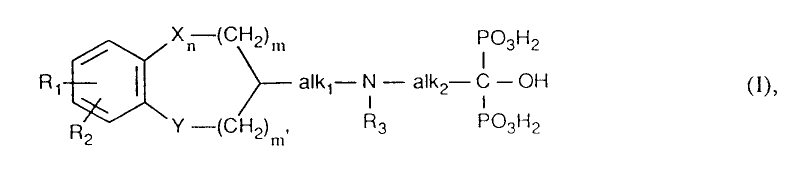
worin R₁ und R₂ unabhängig voneinander Wasserstoff, Niederalkyl, Niederalkoxy, Halogen oder Trifluormethyl bedeuten, R₃ Wasserstoff oder Niederalkyl bedeutet, X und Y unabhängig voneinander Oxy oder Thio darstellen, alk₁ und alk₂ gleiche oder verschiedene Niederalkylenreste bedeuten, n für 0 oder 1 steht und m und m' unabhängig voneinander für 0, 1 oder 2 stehen, wobei die Summe von n, m und m' 1, 2 oder 3 ist, und ihre Salze, Verfahren zur Herstellung der genannten Verbindungen, diese enthaltende pharmazeutische Präparate und ihre Verwendung als Arzneimittelwirkstoffe.

Der durch die Formel 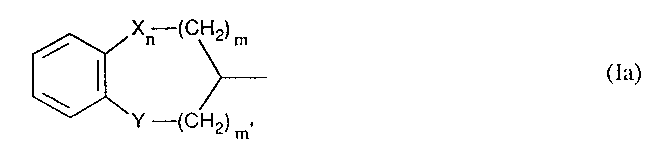
dargestellte Benzoheterocyclylrest ist beispielsweise 2,3-Dihydrobenzofuranyl bzw. 2,3-Dihydrobenzothienyl (Y= Oxy bzw. Thio; n= 0; m= 1 und m'= 0 oder m=0 und m'=1), Chromanyl bzw. Thiochromanyl (Y= Oxy oder Thio, n= 0; m und m'= 1 oder m= 0 und m'= 2 oder m= 2 und m'= 0), 1,4-Benzodioxanyl (X und Y= Oxy; n=1; m=0 und m'=1 oder m=1 und m'=0); 1,4-Benzoxathianyl (X= Oxy und Y= Thio oder X= Thio und Y= Oxy; n=1; m=0 und m'=1 oder m=1 und m'=0); 1,4-Benzodithianyl (X und Y= Thio; n=1; m=0 und m'=1 oder m=1 und m'=0); 2,3,4,5-Tetrahydrobenzoxepinyl bzw. 2,3,4,5-Tetrahydrobenzothiepinyl (Y= Oxy bzw. Thio; n=0; m= 0 und m'= 3 oder m= 1 und m'= 2 oder m= 2 und m'= 1 oder m= 3 und m'= 0), 2,3,4,5-Tetrahydrobenzodioxepinyl (X und Y= Oxy; n= 1; m= 0 und m'= 2 oder m und m' = 1 oder m= 2 und m'= 0), 2,3,4,5-Tetrahydrobenzoxathiepinyl (X= Oxy und Y= Thio oder X= Thio und Y= Oxy; n= 1; m= 0 und m'= 2 oder m und m' = 1 oder m= 2 und m'= 0) oder 2,3,4,5-Tetrahydrobenzodithiepinyl (X und Y= Thio; n= 1; m= 0 und m'= 2 oder m und m' = 1 oder m= 2 und m'= 0). Die genannten Reste können unsubstituiert oder durch einen von Wasserstoff verschiedenen Rest R₁ mono- oder durch gleiche oder verschiedene Reste R₁ und R₂ disubstituiert sein.

Bevorzugt ist 2,3-Dihydrobenzofuranyl, insbesondere 2,3-Dihydrobenzofuran-2-yl, Chromanyl, insbesondere Chroman-3-yl, 1,4-Benzodioxanyl, insbesondere 1,4-Benzodioxan-2-yl, 6-Niederalkyl-, 6-Niederalkoxy- und 7-Halogen-1,4-benzodioxan-2-yl.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Niederalkyl ist C₁-C₇-Alkyl, vorzugsweise C₁-C₄-Alkyl, wie insbesondere Methyl oder in zweiter Linie Äthyl, Propyl, Isopropyl oder Butyl, kann aber auch Isobutyl, Sekundärbutyl, Tertiärbutyl oder eine C₅-C₇-Alkyl-, wie Pentyl-, Hexyl- oder Heptylgruppe sein.

Niederalkylen ist C₁-C₇-Alkylen, insbesondere C₁-C₄-Alkylen, im Falle von alk₁ insbesondere C₁-C₄-Alkylen, wie Methylen, Äthylen, 1,3-Propylen oder 1,4-Butylen, und im Falle von alk₂ insbesondere C₂-C₃-Alkylen, wie Äthylen oder in zweiter Linie 1,3-Propylen.

Niederalkoxy ist C₁-C₇-Alkoxy, vorzugsweise C₁-C₄-Alkoxy, wie Methoxy, Äthoxy, Propyloxy, Isopropyloxy oder Butyloxy , kann aber auch Isobutyloxy, Sekundärbutyloxy, Tertiärbutyloxy oder eine Pentyloxy-, Hexyloxy- oder Heptyloxygruppe sein.

Halogen ist beispielsweise Halogen der Atomnummer bis und mit 35, wie Chlor oder Fluor, ferner Brom.

Salze von Verbindungen der Formel I sind beispielsweise deren Salze mit pharmazeutisch verwendbaren Basen, wie nicht-toxische, von Metallen der Gruppen Ia, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, Kupfer-, Aluminium- oder Zinksalze, ebenso Ammoniumsalze mit Ammoniak oder organischen Aminen oder quaternären Ammoniumbasen, wie gegebenenfalls C-hydroxylierten aliphatischen Aminen, insbesondere Mono-, Di- oder Triniederalkylaminen, z.B. Methyl-, Äthyl- oder Diäthylamin, Mono-, Di- oder Tri-(hydroxyniederalkyl)aminen, wie Äthanol-, Diäthanol- oder Triäthanolamin, Tris-(hydroxymethyl)methylamin oder 2-Hydroxytertiärbutylamin, oder N-(Hydroxyniederalkyl)-N,N-diniederalkyl-aminen bzw. N-(Polyhydroxyniederalkyl)-N-niederalkylaminen, wie 2-(Dimethylamino)äthanol oder D-Glucamin, oder quaternären aliphatischen Ammoniumhydroxiden, z.B. Tetrabutylammoniumhydroxid. Umfasst sind sowohl vollständige als auch partielle Salze, d.h. Salze mit 1, 2, 3 oder 4, vorzugsweise 2, Äquivalente Base pro Mol Säure der Formel I.

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt sind.

Die Verbindungen der Formel I und ihre Salze weisen wertvolle pharmakologische Eigenschaften auf. Insbesondere besitzen sie eine ausgeprägte regulierende Wirkung auf den Calcium- Stoffwechsel von Warmblütern. So bewirken sie an der Ratte eine ausgeprägte Hemmung der Knochenresorption, die sich sowohl in der Versuchsanordnung gemäss Acta Endocrinol. 78, 613-24 (1975) als auch anhand des PTH- induzierten Anstieges des Serumcalciumspiegels nach subkutaner Applikation in Dosen von etwa 0,01 bis etwa 1.0 mg/kg, als auch im TPTX-(Thyroparathyroidectomised)-Rattenmodell anhand der durch Vitamin D₃ ausgelösten experimentellen Hyperkalzämie nach Gabe von Dosen von etwa 0,0005 bis etwa 0,5 mg/kg s.c. und teilweise auch p.o. zeigen lässt. Ebenso wird die durch Walker-256-Tumore induzierte Tumorhyperkalzämie nach peroraler Verabreichung von etwa 1,0 bis etwa 100 mg/kg gehemmt. Ferner zeigen sie in der Adjuvansarthritis der Ratte in der Versuchsanordnung nach Newbold, Brit. J. Pharmacology 21, 127 (1963) sowie nach Kaibara et al., J. Exp. Med. 159, 1388-96 (1984) in Dosen von etwa 0,01 bis etwa 1,0 mg/kg s.c. eine deutliche Hemmung des Fortschreitens chronisch- arthritischer Prozesse. Im Vordergrund stehen dabei die Indikationen tumorinduzierte Hyperkalzämie, Knochenmatastasen und morbus Paget.

Die Verbindungen der Formel I und ihre Salze sind deshalb vorzüglich geeignet als Arzneimittelwirkstoffe für die Behandlung von Erkrankungen, die mit Störungen des Calcium- Stoffwechsels in Verbindung gebracht werden können, insbesondere tumorinduzierter Hyperkalzämie, von Knochenmatastasen und des morbus Paget sowie von entzündlichen Prozessen in Gelenken, degenerativen Prozessen im Gelenkknorpel, von Osteoporosis, Periodontitis, Hyperparathyreoidismus, ferner von Calciumablagerungen in Blutgefässen oder in prothetischen Implantaten.

Die Erfindung betrifft in erster Linie Verbindungen der Formel I, worin R₁ und R₂ unabhängig voneinander Wasserstoff, Niederalkyl, Niederalkoxy, Halogen oder Trifluormethyl bedeuten, R₃ Wasserstoff oder Niederalkyl bedeutet, der durch die Formel Ia dargestellte Benzoheterocyclylrest 2,3-Dihydrobenzofuranyl, 2,3-Dihydrobenzothienyl, Chromanyl, Thiochromanyl, 1,4-Benzodioxanyl, 1,4-Benzoxathianyl, 1,4-Benzodithianyl 2,3,4,5-Tetrahydrobenzoxepinyl, 2,3,4,5-Tetrahydrobenzothiepinyl, 2,3,4,5-Tetrahydrobenzodioxepinyl, 2,3,4,5-Tetrahydrobenzoxathiepinyl oder 2,3,4,5-Tetrahydrobenzodithiepinyl darstellt und alk₁ und alk₂ gleiche oder verschiedene Niederalkylenreste bedeuten, und ihre Salze, insbesondere ihre pharmazeutisch verwendbaren Salze.

Die Erfindung betrifft vor allem Verbindungen der Formel I, worin R₁ und R₂ unabhängig voneinander Wasserstoff, C₁-C₄-Alkyl, wie Methyl, C₁-C₄-Alkoxy, wie Methoxy, oder Halogen der Atomnummer bis und mit 35, wie Chlor, bedeuten, R₃ Wasserstoff oder C₁-C₇-Alkyl, vorzugsweise C₁-C₄-Alkyl, wie insbesondere Methyl oder in zweiter Linie Äthyl, Propyl, Isopropyl oder Butyl, bedeutet, der durch die Formel Ia dargestellte Benzoheterocyclylrest 2,3-Dihydrobenzofuranyl, wie 2,3-Dihydobenzofuran-2-yl, 2,3-Dihydrobenzothienyl, wie 2,3-Dihydrobenzothien-2-yl, Chromanyl, wie Chroman-3-yl, Thiochromanyl, wie Thiochroman-3-yl, 1,4-Benzodioxanyl, wie 1,4-Benzodioxan-2-yl, 1,4-Benzoxathianyl, wie 1,4-Benzoxathian-2-yl oder -3-yl, 1,4-Benzodithianyl, wie 1,4-Benzodithian-2-yl, 2,3,4,5-Tetrahydrobenzoxepinyl, wie 2,3,4,5-Tetrahydrobenzoxepin-2-yl, -3-yl oder -4-yl, 2,3,4,5-Tetrahydrobenzothiepinyl, wie 2,3,4,5-Tetrahydrobenzothiepin-2-yl, -3-yl oder -4-yl, 2,3,4,5-Tetrahydrobenzodioxepinyl, wie 2,3,4,5-Tetrahydrobenzodioxepin-2-yl, -3-yl oder -4-yl, 2,3,4,5-Tetrahydrobenzoxathiepinyl, wie 2,3,4,5-Tetrahydrobenzoxathiepin-2-yl, -3-yl oder -4-yl, oder 2,3,4,5-Tetrahydrobenzodithiepinyl, wie 2,3,4,5-Tetrahydrobenzodithiepin-2-yl, -3-yl oder -4-yl, darstellt und alk₁ und alk₂ gleiche oder verschiedene C₁-C₄-Alkylenreste, im Falle von alk₁ insbesondere C₁-C₄-Alkylen, wie Methylen, Äthylen, 1,3-Propylen oder 1,4-Butylen, und im Falle von alk₂ insbesondere C₂-C₃-Alkylen, wie Äthylen oder in zweiter Linie 1,3-Propylen, bedeuten, und ihre Salze, insbesondere ihre pharmazeutisch verwendbaren Salze.

Die Erfindung betrifft insbesondere Verbindungen der Formel I, worin R₁ und R₂ unabhängig voneinander Wasserstoff, C₁-C₄-Alkyl, wie Methyl, C₁-C₄-Alkoxy, wie Methoxy, oder Halogen der Atomnummer bis und mit 35, wie Chlor, bedeuten, R₃ Wasserstoff oder C₁-C₄-Alkyl, wie insbesondere Methyl oder in zweiter Linie Äthyl oder Propyl, bedeutet, der durch die Formel Ia dargestellte Benzoheterocyclylrest 2,3-Dihydrobenzofuranyl, wie 2,3-Dihydobenzofuran-2-yl, 2,3-Dihydrobenzothienyl, wie 2,3-Dihydrobenzothien-2-yl, Chromanyl, wie Chroman-3-yl, Thiochromanyl, wie Thiochroman-3-yl, 1,4-Benzodioxanyl, wie 1,4-Benzodioxan-2-yl, 1,4-Benzodithianyl, wie 1,4-Benzodithian-2-yl, 2,3,4,5-Tetrahydrobenzoxepinyl, wie 2,3,4,5-Tetrahydrobenzoxepin-2-yl, -3-yl oder -4-yl, 2,3,4,5-Tetrahydrobenzothiepinyl, wie 2,3,4,5-Tetrahydrobenzothiepin-2-yl, -3-yl oder -4-yl, oder 2,3,4,5-Tetrahydrobenzodioxepinyl, wie 2,3,4,5-Tetrahydrobenzodioxepin-2-yl oder -3-yl, 2,3,4,5-Tetrahydrobenzoxathiepinyl, oder 2,3,4,5-Tetrahydrobenzodithiepinyl, wie 2,3,4,5-Tetrahydrobenzodithiepin-2-yl oder -3-yl, darstellt, alk₁ C₁-C₄-Alkylen, wie Methylen, Äthylen oder 1,3-Propylen, bedeutet und und alk₂ C₂-C₃-Alkylen, wie Äthylen, bedeutet, und ihre Salze, insbesondere ihre pharmazeutisch verwendbaren Salze.

Die Erfindung betrifft in allererster Linie Verbindungen der Formel I, worin R₁ Wasserstoff, C₁-C₄-Alkyl, wie Methyl, C₁-C₄-Alkoxy, wie Methoxy, oder Halogen der Atomnummer bis und mit 35, wie Chlor, bedeutet, R₂ Wasserstoff darstellt, R₃ Wasserstoff oder C₁-C₄-Alkyl, wie insbesondere Methyl oder in zweiter Linie Äthyl, bedeutet, der durch die Formel Ia dargestellte Benzoheterocyclylrest 2,3-Dihydrobenzofuranyl, wie 2,3-Dihydrobenzofuran-2-yl, Chromanyl, wie Chroman-3-yl, oder 1,4-Benzodioxanyl, wie 1,4-Benzodioxan-2-yl, bedeutet, alk₁ C₁-C₃-Alkylen, wie Methylen, Äthylen oder 1,3-Propylen, bedeutet und alk₂ Äthylen darstellt, und ihre Salze, insbesondere ihre pharmazeutisch verwendbaren Salze.

Die Erfindung betrifft namentlich die in den Beispielen genannten Verbindungen und ihre Salze, insbesondere ihre pharmazeutisch verwendbaren Salze.

Die Erfindung betrifft ferner ein auf an sich bekannten Methoden beruhendes Verfahren zur Herstellung der erfindungsgemässen Verbindungen. Dieses ist dadurch gekennzeichnet, dass man
a) in einer Verbindung der Formel II 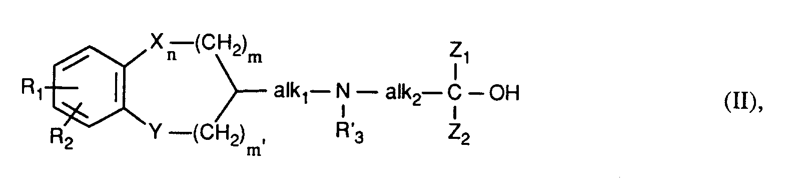 worin R₁, R₂, n, m, m', X, Y, alk₁ und alk₂ die angegebenen Bedeutungen haben, R'₃ eine der genannten Gruppen R₃ oder eine Aminoschutzgruppe R₀, Z₁ eine funktionell abgewandelte und Z₂ eine freie oder funktionell abgewandelte Phosphonogruppe bedeutet, funktionell abgewandeltes Phosphono Z₁ und gegebenenfalls Z₂ in die freie Phosphonogruppe überführt oder
b) Verbindungen der Formeln IIIa und IVa 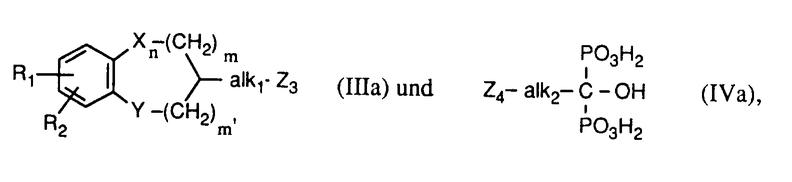 worin R₁, R₂, n, m, m', X, Y, alk₁ und alk₂ die angegebenen Bedeutungen haben, einer der Reste Z₃ und Z₄ eine reaktionsfähige veresterte Hydroxygruppe und der andere eine Gruppe der Formel -N(R'₃)-H darstellt, in der R'₃ eine der genannten Gruppen R₃ oder eine Aminoschutzgruppe R₀ bedeutet, oder deren Salze oder unter reduzierenden Bedingungen Verbindungen der Formeln IIIb und IVb 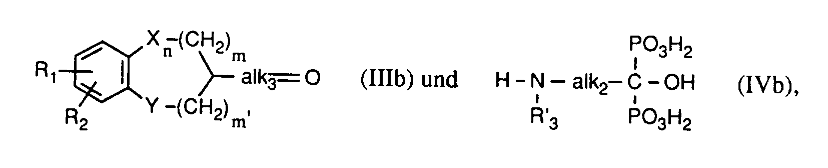 worin alk₃ einen dem Rest alk₁ entsprechenden doppelt gebundenen Rest, d.h. einen Niederalkanylylidenrest, darstellt, R'₃ eine der genannten Gruppen R₃ oder eine Aminoschutzgruppe R₀ bedeutet und R₁, R₂, n, m, m', X, Y, alk₁ und alk₂ die angegebenen Bedeutungen haben, miteinander umsetzt oder
c) eine Verbindung der Formel V 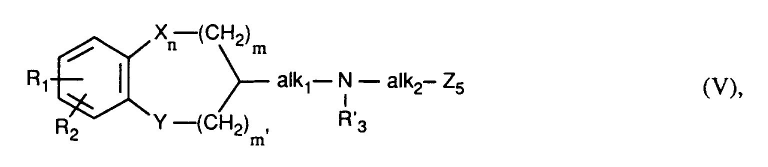 worin Z₅ Carboxy, Carbamoyl oder Cyano und R'₃ eine der genannten Gruppen R₃ oder eine Aminoschutzgruppe R₀ bedeutet und R₁, R₂, n, m, m', X, Y, alk₁ und alk₂ die angegebenen Bedeutungen haben, mit einem Phosphorylierungsmittel umsetzt, das Primärprodukt hydrolysiert und in einem ausgehend von Verbindungen der Formel V, worin Z₅ Cyano oder Carbamoyl ist, erhaltenen Zwischenprodukt der Formel Va 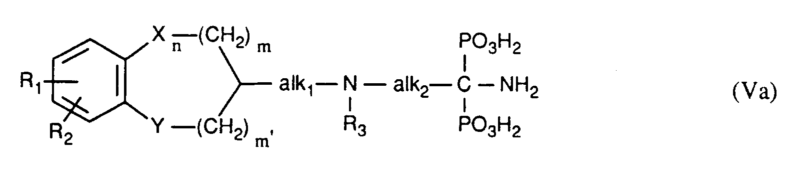 bzw. einem Salz davon die Aminogruppe durch Behandlung mit salpetriger Säure durch Hydroxy ersetzt, jeweils die Aminoschutzgruppe R₀, sofern vorhanden abspaltet und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

Die Durchführung der verfahrensgemässen Reaktionen sowie die Herstellung neuer Ausgangsstoffe bzw. Zwischenprodukte erfolgt in Analogie zur Reaktions- und Bildungsweise bekannter Ausgangsstoffe bzw. Zwischenprodukte. Dabei werden, auch wenn nachstehend nicht ausdrücklich erwähnt, die jeweils üblichen Hilfsmittel, wie Katalysatoren, Kondensations- sowie Solvolysemittel und/oder Lösungs- bzw. Verdünnungsmittel, und Reaktions-, wie Temperatur- und Druckbedingungen, sowie gegebenenfalls Schutzgase verwendet.

Geeignete Aminoschutzgruppen R₀ sind beispielsweise gegebenenfalls substituierte α-Aralkyl-, wie Benzyl-, oder Benzyloxycarbonylgruppen, veresterte oder verätherte Hydroxymethylgruppen, wie Pivaloyloxymethyl, Methoxymethyl, 2-Chloräthoxymethyl oder Benzyloxymethyl, Tetrahydropyranyl oder Triniederalkylsilyl, wie Trimethylsilyl. Die Schutzgruppe wird beispielsweise durch Umsetzung der zu schützenden Verbindung mit einem entsprechenden Halogenderivat bzw. mit Chlorjodmethan (Cl-CH₂I), einem Alkalimetall-, z.B. Natriumpivalat, -methanolat, -1,2-dichloräthanolat oder -benzylalkoholat bzw. mit Dihydropyran, eingeführt.

Gemäss der Verfahrensvariante a) in Phosphono zu überführende funktionell abgewandelte Phosphonogruppen liegen beispielsweise in einer Esterform, insbesondere einer Diesterform der Formel -P(=O)(OR)₂ (IIa), vor, worin OR veräthertes Hydroxy, insbesondere Niederalkoxy, Niederalkanoyloxyniederalkoxy oder eine gegebenenfalls durch Niederalkyl, Niederalkoxy, Halogen, Trifluormethyl und/oder Hydroxy substituierte Phenoxy- oder α-Phenylniederalkoxygruppe oder Silyloxy, wie Triniederalkylsilyloxy, bedeutet.

Die Überführung von funktionell abgewandelten in freie Phosphonogruppen erfolgt in üblicher Weise, wie durch Hydrolyse, beispielsweise in Gegenwart einer Mineralsäure, wie Salz- oder Schwefelsäure, bei etwa 80°C bis etwa 110°C, z.B. in der Siedehitze, oder durch Umsetzung mit einem Triniederalkylhalogensilan, z.B. mit Trimethylchlorsilan oder insbesondere Trimethyljodsilan oder Trimethylbromsilan, vorzugsweise in Methylenchlorid im Temperaturbereich von etwa 0°C bis etwa 40°C, und anschliessende Behandlung mit Wasser. α-Phenylniederalkylester können ferner durch Hydrogenolyse, beispielsweise Umsetzung mit Wasserstoff in Gegenwart eines Hydrierungskatalysators, wie eines Nickel- oder Edelmetallkatalysators, z.B. von Palladium auf Kohle, vorzugsweise in einem Niederalkanol unter normalen Temperatur- und Druckbedingungen, in Verbindungen der Formel I überführt werden.

Die Ausgangsstoffe der Formel II können beispielsweise hergestellt werden, indem man eine Verbindung der Formel IIb 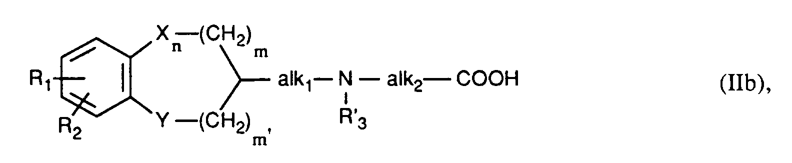
wobei R₁, R₂, n, m, m', X, Y, alk₁ und alk₂ die angegebenen Bedeutungen haben und R'₃ eine der genannten Gruppen R₃ oder eine Aminoschutzgruppe R₀ bedeutet, oder das Anhydrid oder Säurechlorid derselben, beispielsweise bei 0°C bis etwa 60°C, mit einem entsprechenden Phosphorigsäuretriester der Formel P(OR)₃ (IIc)zu einer Verbindung der Formel (IId) 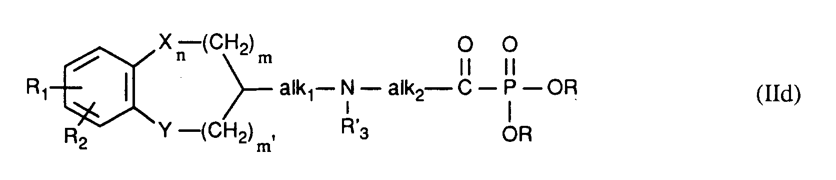
kondensiert und diese mit einem Phosphorigsäurediester der Formel H-P(=O)(OR)₂ (IIe) bzw. P(OH)(OR)₂ (IIf) in Gegenwart eines Diniederalkylamins, z.B. von Diäthylamin, oder eines Alkalimetallniederalkanolates, z.B. von Natriummethanolat, zur entsprechenden Verbindung der Formel IIg 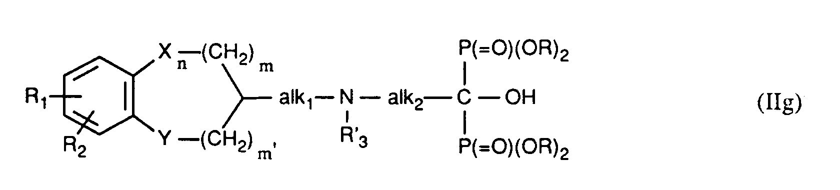
weiterumsetzt, gegebenenfalls die Aminoschutzgruppe R₀ abspaltet und/oder gewünschtenfalls einen von von Wasserstoff verschiedenen Rest R₃ einführt. z.B. wie nachstehend unter b) beschrieben.

Ausgangsstoffe der Formel IIb können, soweit sie nicht bekannt sind, beispielsweise hergestellt werden, indem man eine entsprechende Verbindung der Formel (IIh) 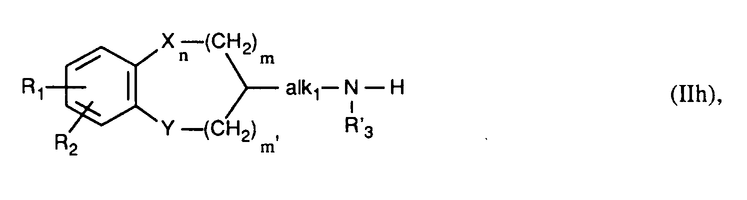
worin R'₃ eine Gruppe R₃ oder eine Aminoschutzgruppe R₀ bedeutet, mit einer Verbindung der Formel Z₆-alk₂-COOR (IIi), worin Z₆ Halogen, wie Brom, ist oder zur Herstellung von Verbindungen IIb, worin alk₂ 1,2-Niederalkylen, z.B. Äthylen, bedeutet, einer Verbindung der Formel alk₀-COOR (IIj), worin alk₀ Niederalk-1-enyl bedeutet, umsetzt, jeweils den erhaltenen Ester zur Säure hydrolysiert, eine gegebenenfalls vorhandene Aminoschutzgruppe abspaltet und erforderlichen- oder gewünschtenfalls die freigesetzte Aminogruppe niederalkyliert.

Gemäss der Verfahrensvariante b) zu verwendende reaktive Ester IIIa bzw. IVa weisen als reaktionsfähige veresterte Hydroxygruppen beispielsweise ein Halogen-, wie Chlor-, Brom- oder Jodatom, oder eine Sulfonyloxygruppe, z.B. Methansulfonyloxy oder p-Toluolsulfonyloxy, auf.

Die Umsetzung mit den genannten reaktiven Estern erfolgt beispielsweise in Gegenwart einer Base, wie eines Alkalimetall- oder Erdalkalimetallhydroxides, z.B. von Natriumhydoxid, oder eines quaternären Ammoniumhydroxides, z.B. von Tetrabutylammoniumhydroxid, vorteilhaft in Gegenwart eines Lösungs- oder Verdünnungsmittels, z.B. eines Niederalkanols, Diniederalkylketons oder cycloaliphatischen Äthers, z.B. von Isopropanol, Methyläthylketon, Dioxan oder Tetrahydrofuran.

Die Umsetzung von Oxoverbindungen IIIb mit Aminoalkandiphosphonsäuren IVb wird beispielsweise in Gegenwart eines Alkalimetallborhydrides, z.B. von Natriumcyanoborhydrid, oder insbesondere durch Behandeln mit Ameisensäure durchgeführt.

Die Ausgangsstoffe der Formel IVa können beispielsweise hergestellt werden, indem man eine Verbindung der Formel Z₄-alk₂-Z₅ (IVc), worin Z₄ die unter der Formel IVa und Z₅ die unter der Formel V angegebene Bedeutung hat, wobei Z₄ vorzugsweise Halogen, wie Brom, ist, oder zur Herstellung von Verbindungen der Formel V, worin alk₂ 1,2-Niederalkylen, z.B. Äthylen, bedeutet, mit einer Verbindung der Formel alk₀- Z₅ (IVd), worin alk₀ einen Niederalk-1-enylrest bedeutet, in üblicher Weise, beispielsweise in Chlorbenzol, mit phosphoriger Säure und Phosphortrichlorid bzw. mit Phosphorsäure und einem Überschuss an Phosphortribromid umsetzt und anschliessend hydrolytisch aufarbeitet. Ausgangsstoffe IVb können in analoger Weise durch Umsetzung von Verbindungen der Formel R₀-N(R'₃)-alk₂-Z₅ (IVe) mit phosphoriger Säure und Phosphortrichlorid hergestellt werden, wobei R₀ eine übliche Aminoschutzgruppe bedeutet.

Als Phosphorylierungsmittel für die Verfahrensvariante c) kommen beispielsweise Phosphortrioxid, Phosphortrihalogenide im Gemisch mit phosphoriger Säure oder Phosphorsäure, Phosphoroxychlorid oder Phosphorpentachlorid bzw. Phosphortrichlorid im Gemisch mit Chlor in Betracht. Bevorzugt ist Phosphortrioxid, welches vorzugsweise durch Umsetzung von Phosphortrichlorid mit phosphoriger Säure bzw. die Phosphorigsäurekomponente vorzugsweise durch Reaktion mit einem Überschuss von Phosphortrichlorid mit wasserhaltiger Phosphorsäure, z.B. mit handelsüblicher etwa 75%-iger bis 95%-iger, vorzugsweise etwa 85%-iger, Phosphorsäure, *in situ* gebildet wird. Die Umsetzung wird vorteilhaft unter Erwärmen, z.B. auf etwa 70 bis 120°C, in einem geeigneten Lösungsmittel, wie Tetrachloräthan, Trichloräthan, Chlorbenzol, Chlortoluol oder Paraffinöl, und unter hydrolytischer Aufarbeitung durchgeführt.

Die Behandlung von Zwischenprodukten der Formel Va mit salpetriger Säure erfolgt in üblicher Weise unter Freisetzung derselben in wässriger Lösung aus einem ihrer Salze, z.B. aus Natriumnitrit, durch Säurebehandlung, z.B. Einwirkung von Salzsäure, wobei intermediär ein entsprechendes instabiles Diazoniumsalz, z.B. -chlorid, gebildet wird, das unter Einführung der α-Hydroxygruppe Stickstoff abspaltet.

Die Ausgangsstoffe der Formel V können, soweit sie nicht bekannt sind, beispielsweise hergestellt werden, indem man eine entsprechende Verbindung der Formel(IIh) 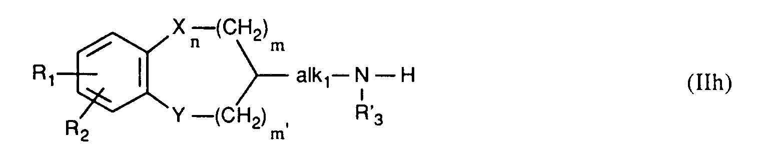
mit einer Verbindung der Formel Z₄-alk₂-Z₅(IVc), worin Z₄ die unter der Formel IVa und Z₅ die unter der Formel V angegebene Bedeutung hat, wobei Z₄ vorzugsweise Halogen, wie Brom, ist, oder zur Herstellung von Verbindungen der Formel V, worin alk₂ 1,2-Niederalkylen, z.B. Äthylen, bedeutet, mit einer Verbindung der Formel alk₀- Z₅ (IVd), worin alk₀ einen Niederalk-1-enylrest bedeutet, umsetzt, jeweils die Aminoschutzgruppe, sofern vorhanden, abspaltet und gewünschtenfalls jeweils das erhaltene Primärprodukt zur Säure hydrolysiert.

Verfahrensgemäss erhältliche Verbindungen können in üblicher Weise in andere Verbindungen der Formel I überführt werden.

So kann man in Verbindungen der Formel I, worin R₃ Wasserstoff bedeutet, in üblicher Weise durch Umsetzung mit einem reaktiven Ester der Formel R₃-Z₆ (IX), worin R₃ Niederalkyl und Z₆ reaktionsfähiges verestertes Hydroxy, beispielsweise ein Halogen-, wie Chlor-, Brom- oder Jodatom, oder eine Sulfonyloxygruppe, z.B. Methansulfonyloxy oder p-Toluolsulfonyloxy, bedeutet, oder durch Umsetzung mit einen Niederalkanal oder Diniederalkylketon der Formel R₃=O (IXa) unter reduzierenden Bedingungen, Niederalkyl R₃ einführen.

Die Umsetzung mit den genannten reaktiven Estern (IX) erfolgt beispielsweise in Gegenwart einer Base, wie eines Alkalimetall- oder Erdalkalimetallhydroxides, z.B. von Natriumhydroxid, oder eines quaternären Ammoniumhydroxides, z.B. von Tetrabutylammoniumhydroxid, vorteilhaft in Gegenwart eines Lösungs- oder Verdünnungsmittels, z.B. eines Niederalkanols, Diniederalkylketons oder cycloaliphatischen Äthers, z.B. von Isopropanol, Methyläthylketon, Dioxan oder Tetrahydrofuran. Die Umsetzung mit Oxoverbindungen (IXa) wird beispielsweise in Gegenwart eines Alkalimetallborhydrides, z.B. von Natriumcyanoborhydrid, oder insbesondere durch Behandeln mit Ameisensäure durchgeführt. In einer bevorzugten Ausführungsform kann man eine entsprechende Verbindung der Formel Ia unter reduzierenden Bedingungen mit einem Niederalkanal, beispielsweise mit Formaldehyd, und Ameisensäure, durch einen Niederalkylrest R₃ substituieren.

Auch kann man in den Benzoteil des Benzoheterocyclylrestes von Verbindungen der Formel I von Wasserstoff verschiedene Gruppen R₁ und/oder R₂ einführen, Niederalkyl beispielsweise durch Umsetzung mit einem Niederalkylhalogenid in Gegenwart von Aluminiumtrichlorid, Niederalkoxy beispielsweise durch Nitrieren, Reduktion der Nitrogruppe zur Aminogruppe, Diazotieren derselben und Behandeln des gebildeten Diazoniumsalzes mit dem entsprechenden Niederalkanol unter Erwärmen und Halogen beispielsweise durch Behandeln mit Chlor oder Brom, vorteilhaft in Gegenwart einer Lewissäure, z.B. von Eisen-III-chlorid. Man kann aber auch Halogen durch Trifluormethyl ersetzen, beispielsweise durch Behandeln mit Trifluorjodmethan in Gegenwart von Kupferpulver oder Kupfer-I-jodid.

Die neuen Verbindungen können, je nach Wahl der Ausgangsstoffe und Arbeitsweisen, in Form eines der möglichen Isomeren, z.B. je nach Anzahl der asymmetrischen Kohlenstoffatome als optische Isomere, wie in Form eines Enantiomeren, wie Antipoden, bzw. Diastereomeren, oder als Gemische derselben, wie Enantiomerengemische, z.B. Racemate, Diastereomerengemische oder Racematgemische, vorliegen.

Erhaltene Diastereomerengemische und Racematgemische können auf Grund der physikalisch-chemischen Unterschiede der Bestandteile in bekannter Weise in die reinen Diastereomeren bzw. Racemate aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung einer Verbindung der Formel I oder eines Anhydrides davon mit einer optisch aktiven Base bzw. mit einem optisch aktiven Alkohols und Trennung der erhaltenen diastereomeren Ester, z.B. auf Grund ihrer verschieden Löslichkeiten, in die Diastereomeren, aus denen die Enantiomeren durch Einwirkung geeigneter Mittel freigesetzt werden können. Racemate der Formel I können auch durch Umsetzung mit einer optisch aktiven Base in Gemische der diastereomeren Salze, und Trennung derselben in die Diastereomeren, aus denen die Enantiomeren in der jeweils üblichen Weise freigesetzt werden können, gespalten werden.

Für diesen Zweck übliche optisch aktive Basen sind z.B. optisch aktive Alkaloide, wie Chinin, Chinchonin, Brucin und dergl., oder insbesondere α-Phenyläthylamin.

Ferner können erhaltene salzbildende Verbindungen in an sich bekannter Weise in Salze überführt werden, z.B. durch Umsetzung einer Lösung der freien Verbindung in einem geeigneten Lösungsmittel oder Lösungsmittelgemisch mit einer entsprechenden Base oder mit einem geeigneten Ionenaustauscher.

Erhaltene Salze können in an sich bekannter Weise in die freien Verbindungen umgewandelt werden, z.B. durch Behandeln mit einer Säure, wie einer Mineralsäure, z.B. mit Salzsäure.

Erhaltene Salze können in an sich bekannter Weise in andere Salze überführt werden, z.B. durch Behandeln mit einer geeigneten Base, wie Natriumhydroxid oder Kaliumhydroxid, Ammoniak oder einem geeigneten Amin.

Die Verbindungen der Formel I, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeichneten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Die neuen Verbindungen der Formel I können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Lactose, Dextrose, Saccharose, Mannit, Sorbit, Cellulose und/oder Schmiermitteln, z.B. Kieselerde, Talk, Stearinsäure oder Salzen davon, wie Magnesium- oder Calciumstearat, und/oder Polyäthylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, z.B. Natriumalginat, und/oder Brausemischungen, oder Absorptionsmittel, Farbstoffe, Geschmacksstoffe und Süssmittel aufweisen. Ferner kann man die neuen Verbindungen der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservierungs-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. Mittels konventioneller Misch-, Granulier-, Dragier-, Lösungs- oder Lyophilisierungsverfahren hergestellt und enthalten von etwa 0,1% bis 100%, insbesondere von etwa 1% bis etwa 50%, Lyophilisate bis etwa 100% des Aktivstoffes.

Zur parenteralen Verabreichung eignen sich in erster Linie wässrige Lösungen des Wirkstoffes in wasserlöslicher Form, z.B. in Form eines wasserlöslichen pharmazeutisch verwendbaren Salzes, ferner Suspensionen das Wirkstoffes, wie ölige Injektionssuspensionen, wobei man geeignete lipophile Lösungsmittel oder Vehikel, wie fette Öle, z.B. Sesamöl, oder synthetische Fettsäureester, z.B. Äthyloleat, ferner Triglyceride, verwendet oder wässrige Injektionssuspensionen, welche viskositätserhöhende Stoffe, z.B. Natriumcarboxymethylcellulose, Sorbit und/oder Dextran, und gegebenenfalls Stabilisatoren enthalten.

Die Erfindung betrifft weiterhin die Verwendung von Verbindungen der Formel I, zur Behandlung von auf Störungen des Calciumstoffwechsels zurückzuführenden Erkrankungen, vorzugsweise durch Bereitstellung von pharmazeutischen Präparaten. Die Dosierung der erfindungsgemässen Verbindung der Formel I kann von verschiedenen Faktoren, wie Applikationsweise, Spezies, Alter und/oder individuellem Zustand abhängen. Einzeldosen enthalten beispielsweise von etwa 0,01 bis etwa 0,1 mg, vorzugsweise 0,02 bis 0,08 mg bei parenteraler und etwa 0,2 bis etwa 2,5 mg, vorzugsweise 0,3 bis 1,5 mg, bei oraler Gabe jeweils pro Kilogramm Körpergewicht. Die bevorzugten Einzeldosen betragen somit etwa 0.5 bis 5.0 mg bei parenteraler und etwa 10 bis 100 mg bei oraler Applikation. Die täglich zu verabreichenden Dosen liegen bei oraler Applikation zwischen etwa 0,25 und etwa 10 mg/kg und für Warmblüter mit einem Körpergewicht von etwa 70 kg vorzugsweise zwischen etwa 20 mg und etwa 500 mg.

Die nachfolgenden Beispiele dienen zur Illustration der Erfindung; Temperaturen sind in Celsiusgraden, Drucke in mbar angegeben.

Beispiel 1: 5,0 g (0,0175 Mol) 3-{N-[2-(Chroman-3-yl)äthyl]amino}propionsäure-hydrochlorid werden mit 2,4 ml 85%-iger Phosphorsäure und 15 ml Chlorbenzol unter Rühren und Rückfluss auf 100° erhitzt. Dann werden 4,6 ml Phosphortrichlorid zugetropft, wobei Gasentwicklung eintritt. Das Reaktionsgemisch scheidet im Laufe von 30 Minuten eine dicke Masse ab. Man erhitzt noch 2 Stunden auf 100° und dekantiert dann das überstehende Chlorbenzol ab. Die zurückbleibende zähe Masse wird mit 40 ml 4n-Salzsäure 3 Stunden unter Rühren und Rückfluss zum Sieden erhitzt. Man filtriert heiss unter Kohlezusatz und engt das Filtrat unter vermindertem Druck zur Sirupdicke ein. Auf Zusatz von Aceton kristallisiert die 3-{N-[2-{Chroman-3-yl)äthyl]amino}-1-hydroxypropan-1,1-diphosphonsäurevom Smp. 187-192° (Zers.).

Das Ausgangsmaterial kann z.B. folgendermassen hergestellt werden:
3,0 g (0,01 Mol) 3-{N-[2-(Chroman-3-yl)äthyl]amino}propionsäuremethylesterhydrochlorid werden mit 20 ml 4n-Salzsäure 4 Stunden unter Rühren zum Sieden erhitzt. Dann wird unter vermindertem Druck eingedampft und der Rückstand mit Aceton verrührt. Man erhält das 3-{N-[2-(Chroman-3-yl)äthyl]amino}propionsäure-hydrochlorid vom Smp. 186-188°.

Beispiel 2: 4,1 g (0,010 Mol) 3-{N-[2-(Chroman-3-yl)äthyl]amino}-1-hydroxy-propan-1,1-diphosphonsäure werden mit 20 ml n-Natriumhydroxidlösung und 4,0 ml einer 37%-igen wässrigen Formaldehydlösung verrührt und portionsweise mit 1,0 g Natriumcyanoborhydrid versetzt. Nach 5 Stunden Rühren bei 20° werden 4,0 ml 36%-ige Salzsäure zugetropft. Nach Einengen unter vermindertem Druck auf das halbe Volumen und Abkühlen im Eisbad kristallisiert die 3-{N-[2-(Chroman-3-yl)äthyl)-N-methylamino}-1-hydroxy-propan-1,1-diphosphonsäure vom Smp. 225-226° (Zers.) aus. Sie wird abgesaugt und unter vermindertem Druck getrocknet.

Beispiel 3: In analoger Weise wie in den Beispielen 1 und 2 beschrieben kann man auch die folgenden Verbindungen herstellen:
3-[N-(6-Methoxy-1,4-benzodioxan-2-yl)methyl-N-methyl-amino]-1-hydroxy-propan-1,1-diphosphonsäure;
3-[N-(7-Chlor-1,4-benzodioxan-2-yl)methyl-N-methyl-amino]-1-hydroxy-propan-1,1-diphosphonsäure und
3-[N-(2,3-Dihydrobenzothien-2-yl)methyl-N-methyl-amino]-1-hydroxy-propan-1,1-diphosphonsäure.

Beispiel 4: In analoger Weise wie in Beispiel 1 beschrieben erhält man ausgehend von 3-{N-[2-(Chroman-3-yl)äthyl]-N-methyl-amino}-propionsäure-hydrochlorid die 3-{N-[2-(Chroman-3-yl)äthyl]-N-methyl-amino}-1-hydroxy-propan-1,1-diphosphonsäure vom Smp. 225-226° (identisch mit dem Produkt gemäss Beispiel 2).

Das Ausgangsmaterial kann z.B. folgendermassen hergestellt werden:
3,0 g (0,01 Mol) 3-{N-[2-(Chroman-3-yl)äthyl]amino}propionsäuremethylesterhydrochlorid werden unter Rühren in 30 ml Acetonitril suspendiert und mit 4,0 ml (0,05 Mol) 37%-igem Formaldehyd in Wasser versetzt. Dann werden 0,84 g (0,01 Mol) Natriumhydrogencarbonat zugegeben, wonach unter fortgesetztem Rühren eine klare Lösung erhalten wird. Diese wird portionsweise mit 1,0 g Natriumcyanoborhydrid versetzt, worauf man den pH-Wert der Suspension durch tropfenweise Zugabe von Eisessig auf pH 7 stellt. Man lässt 1 Stunde bei Raumtemperatur nachrühren, destilliert unter vermindertem Druck das Lösungsmittel ab und verteilt den öligen Rückstand zwischen 50 ml Diäthyläther und 15 ml n-Natriumhydrogencarbonatlösung. Die organische Phase wird abgetrennt, über Natriumsulfat getrocknet und eingedampft. Chromatographische Reinigung an einer Kieselgelsäule (2 cm ⌀, 30 cm Länge, Kieselgel® Merck 60) mit Toluol/Äthanol (9:1 Vol.-Teile) ergibt den 3-{N-[2-(Chroman-3-yl)äthyl]-N-methyl-amino}-propionsäuremethylester als Öl. Aus diesem erhält man in analoger Weise wie in Beispiel 1 beschrieben durch Erhitzen mit 20 ml 4n-Salzsäure das 3-{N-[2-(Chroman-3-yl)äthyl]-N-methyl-amino}-propionsäure-hydrochlorid vom Smp. 169-170°.

Beispiel 5: In analoger Weise wie in Beispiel 1 beschrieben erhält man ausgehend von 3,0 g (0,01 Mol) 3-{N-[2-(Chroman-2-yl)äthyl]-N-methyl-amino}-propionsäuremethylester-hydrochlorid über 3-{N-[2-(Chroman-2-yl)äthyl]-N-methyl-amino}propionsäure-hydrochlorid vom Smp. 130-132° die 3-{N-(2-(Chroman-2-yl)äthyl]-N-methyl-amino}-1-hydroxy-propan-1,1-diphosphonsäure vom Smp. 151-155° (Zers.).

Beispiel 6: In analoger Weise wie in Beispiel 1 beschrieben erhält man ausgehend von 2,8 g ((0,01 Mol) 3-[N-(6-Methylbenzo-1,4-dioxan-2-ylmethyl)amino]-propionsäureäthylester nach 4-stündigem Siedenlassen mit 20 ml 4n-Salzsäure am Rückfluss 3-[N-(6-Methylbenzo-1,4-dioxan-2-ylmethyl)amino]-propionsäure-hydrochlorid vom Smp 154-156° und aus diesem die 3-[N-(6-Methylbenzo-1,4-dioxan-2-ylmethyl)amino]-1-hydroxy-propan-1,1-diphosphonsäure vom Smp. 183-184° (Zers.).

Das Ausgangsmaterial kann z.B. folgendermassen hergestellt werden:
7,2 g (0,04 Mol) 6-Methylbenzo-1,4-dioxan-2-ylmethylamin werden mit 4,4 ml (0,04 Mol) Acrylsäureäthylester versetzt und 60 Stunden bei Raumtemperatur stehengelassen. Man erhält rohen 3-[N-(6-Methylbenzo-1,4-dioxan-2-ylmethyl)amino]propionsäureäthylester, der ohne weitere Reinigung eingesetzt werden kann.

Beispiel 7: In analoger Weise wie in Beispiel 1 beschrieben erhält man ausgehend von 3-[N-(Benzo-1,4-dioxan-2-ylmethyl)-N-methyl-amino]-propionsäure-hydrochlorid die 3-[N-(Benzo-1,4-dioxan-2-ylmethyl)-N-methyl-amino]-1-hydroxy-propan- 1,1-diphosphonsäure vom Smp. 176-180° (Zers.).

Beispiel 8: In analoger Weise wie in Beispiel 1 beschrieben erhält man ausgehend von 2,8 g (0,01 Mol) 3-[N-(5-Methoxy-2,3-dihydro-benzofuran-2-yl)methylamino]-propionsäureäthylester durch Erhitzen mit Salzsäure, Eindampfen und Anreiben mit Aceton 3-[N-(5-Methoxy-2,3-dihydro-benzofuran-2-yl)methylamino]-propionsäurehydrochlorid vom Smp. 150-154° und aus diesem die 3-[N-(5-Methoxy-2,3-dihydrobenzofuran-2-yl)methylamino]-1-hydroxy-propan-1,1-diphosphonsäure vom Smp. 157-161° (Zers.).

Das Ausgangsmaterial kann z.B. folgendermassen hergestellt werden:
6,5 g (0,04 Mol) 5-Methoxy-2,3-dihydro-benzofuran-2-ylmethylamin werden mit 4,4 ml (0,04 Mol) Acrylsäureäthylester versetzt und 60 Stunden bei Raumtemperatur stehengelassen. Man erhält 3-[N-(5-Methoxy-2,3-dihydro-benzofuran-2-yl)methylamino]-propionsäureäthylester als gelbliches Öl.

Beispiel 9: In analoger Weise wie in Beispiel 2 beschrieben erhält man ausgehend von 3-[N-(6-Methylbenzo-1,4-dioxan-2-yl)methylamino]-1-hydroxy-propan-1,1-diphosphonsäure die 3-[N-(6-Methylbenzo-1,4-dioxan-2-ylmethyl)-N-methyl-amino]-1-hydroxypropan-1,1-diphosphonsäure vom Smp. 175-178° (Zers.).

Beispiel 10: In analoger Weise wie in Beispiel 1 beschrieben erhält man ausgehend von 7,0 g (0,022 Mol) 3-[N-(6-Chlorbenzo-1,4-dioxan-2-ylmethyl)-N-methyl-amino] -propionsäureäthylester durch Erhitzen mit Salzsäure, Eindampfen und Anreiben mit Aceton 3-[N-(6-Chlorbenzo-1,4-dioxan-2-ylmethyl)-N-methyl-amino]-propionsäurehydrochlorid vom Smp. 122-125° und aus diesem die 3-[N-(6-Chlorbenzo-1,4-dioxan-2-ylmethyl)-N-methyl-amino]-1-hydroxy-propan-1,1-diphosphonsäure vom Smp. 174-176° (Zers.).

Das Ausgangsmaterial kann z.B. folgendermassen hergestellt werden:
14,8 g (0,0675 Mol) 6-Chlor-2-chlormethyl-benzo-1,4-dioxan und 31,0 g Methylamin werden im Druckrohr 24 Stunden auf 120° erhitzt. Nach Abdampfen des überschüssigen Methylamins und Destillation unter vermindertem Druck erhält man das 6-Chlor-2-methylaminomethyl-benzo-1,4-dioxan vom Kp. 70-73° (bei 0,001 mbar).

5,0 g (0,023 Mol) 6-Chlor-2-methylaminomethyl-benzo-1,4-dioxan werden mit 3,3 ml (0,03 Mol) Acrylsäureäthylester versetzt und 60 Stunden bei Raumtemperatur stehengelassen. Man erhält rohen 3-[N-(6-Chlorbenzo-1,4-dioxan-2-ylmethyl)-N-methyl-amino]-propionsäureäthylester, der ohne weitere Reinigung eingesetzt werden kann.

Beispiel 11: Tabletten, enthaltend je 50 mg 3-{N-[2-(Chroman-3-yl)äthyl)-N-methylamino}-1-hydroxy-propan-1,1-diphosphonsäure oder ein Salz, z.B. das Dinatriumsalz, davon können wie folgt hergestellt werden:

### Zusammensetzung (10000 Tabletten)

| | |
|---|---|
| Wirkstoff | 500,0 g |
| Lactose | 500,0 g |
| Kartoffelstärke | 325,0 g |
| Gelatine | 8,0 g |
| Talk | 60,0 g |
| Magnesiumstearat | 10,0 g |
| Siliciumdioxid (hochdispers) | 20,0 g |
| Äthanol | q.s. |

Der Wirkstoff wird mit der Lactose und 292 g Kartoffelstärke vermischt, die Mischung mit einer äthanolischen Lösung der Gelatine befeuchtet und durch ein Sieb granuliert. Nach dem Trocknen mischt man den Rest der Kartoffelstärke, der Talk, das Magnesiumstearat und das Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 12: Lacktabletten, enthaltend je 100 mg 3-{N-[2-(Chroman-3-yl)äthyl)-N-methyl-amino}-1-hydroxy-propan-1,1-diphosphonsäure oder ein Salz, z.B. das Dinatriumsalz, davon können wie folgt hergestellt werden:

### Zusammensetzung (für 1000 Lacktabletten)

| | |
|---|---|
| Wirkstoff | 100,0 g |
| Lactose | 100,0 g |
| Maisstärke | 70,0 g |
| Talk | 8,5 g |
| Calciumstearat | 1,5 g |
| Hydroxypropylose | 2,36 g |
| Schellack | 0,64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 280 mg) verpresst und diese mit einer Lösung der Hydroxypropyl-methylcellulose und des Schellacks in Methylenchlorid lackiert; Endgewicht der Lacktablette: 283 mg.

Beispiel 13: Gelatinesteckkapseln, enthaltend 100 mg Wirkstoff, z.B. 3-{N-[2-(Chroman-3-yl)äthyl)N-methyl-amino}-1-hydroxy-propan-1,1-diphosphonsäure oder ein Salz, z.B. das Dinatriumsalz, davon, können z.B. folgendermassen hergestellt werden:

### Zusammensetzung (für 1000 Kapseln)

| | |
|---|---|
| Wirkstoff | 100,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird die mikrokristalline Zellulose durch ein Sieb mit einer Maschenweite von 0,9 mm hinzugesiebt. Dann wird erneut 10 Minuten innig vermischt. Zuletzt wird das Magnesiumstearat durch ein Sieb mit einer Maschenweite von 0,8 mm hinzugesiebt. Nach 3-minütigem weiteren Mischen werden je 390 mg der erhaltenen Formulierung in Gelatinesteckkapseln der Grösse 0 abgefüllt.

Beispiel 14: Eine 0,2%-ige Injektions- oder Infusionslösung von 3-{N-[2-(Chroman-3-yl)äthyl)-N-methyl-amino}-1-hydroxy-propan-1,1-diphosphonsäure oder eines ihrer Salze, z.B. ihr Dinatriumsalz, kann beispielsweise folgendermassen hergestellt werden:

### Zusammensetzung (für 1000 Ampullen)

| | |
|---|---|
| Wirkstoff | 5,0 g |
| Natriumchlorid | 22,5 g |
| Phosphatpuffer pH=7.4 | 300,0 g |
| entmineralisiertes Wasser | ad 2500,0 ml |

Der Wirkstoff wird in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glas- oder Kunststoffampullen abgefüllt, die dann je 2,0 bzw. 5,0 mg Wirkstoff enthalten.

Beispiel 15: In analoger Weise wie in den Beispielen 11 bis 14 beschrieben kann man auch pharmazeutische Präparate, enthaltend eine andere Verbindung der Formel I gemäss einem der Beispiele 1 bis 10 herstellen.

## Patentansprüche (Patentansprüche für folgende(n) Vertragsstaat(en): AT, BE, CH, DE, DK, FR, GB, IT, LI, LU, NL, SE)

1. Benzoheterocyclylalkylaminoalkandiphosphonsäuren der Formel I 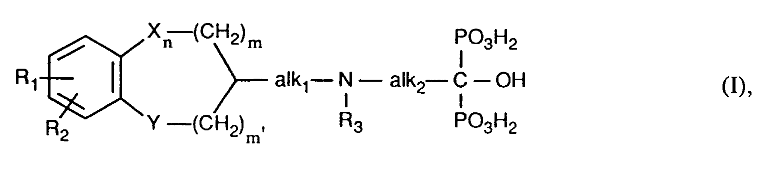 worin R₁ und R₂ unabhängig voneinander Wasserstoff, C₁-C₇-Alkyl, C₁-C₇-Alkoxy, Halogen oder Trifluormethyl bedeuten, R₃ Wasserstoff oder C₁-C₇-Alkyl bedeutet, X und Y unabhängig voneinander Oxy oder Thio darstellen, alk₁ und alk₂ gleiche oder verschiedene C₁-C₇-Alkylenreste bedeuten, n für 0 oder 1 steht und m und m' unabhängig voneinander für 0, 1 oder 2 stehen, wobei die Summe von n, m und m' 1, 2 oder 3 ist, und ihre Salze.

2. Verbindungen gemäss Anspruch 1 der Formel I, worin R₁ und R₂ unabhängig voneinander Wasserstoff, C₁-C₇-Alkyl, C₁-C₇-Alkoxy, Halogen oder Trifluormethyl bedeuten, R₃ Wasserstoff oder C₁-C₇-Alkyl bedeutet, der durch die Formel 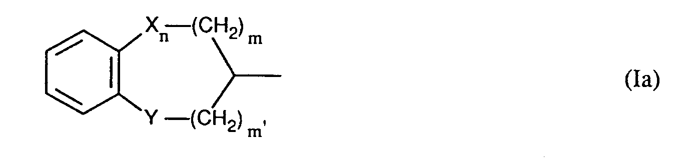 dargestellte Benzoheterocyclylrest 2,3-Dihydrobenzofuranyl, 2,3-Dihydrobenzothienyl, Chromanyl, Thiochromanyl, 1,4-Benzodioxanyl, 1,4-Benzoxathianyl, 1,4-Benzodithianyl 2,3,4,5-Tetrahydrobenzoxepinyl, 2,3,4,5-Tetrahydrobenzothiepinyl, 2,3,4,5-Tetrahydrobenzodioxepinyl, 2,3,4,5-Tetrahydrobenzoxathiepinyl oder 2,3,4,5-Tetrahydrobenzodithiepinyl darstellt und alk₁ und alk₂ gleiche oder verschiedene C₁-C₇-Alkylenreste bedeuten, und ihre Salze, wobei die genannten Benzoheterocyclylreste unsubstituiert oder durch einen von Wasserstoff verschiedenen Rest R₁ mono- oder durch gleiche oder verschiedene Reste R₁ und R₂ disubstituiert sind.

3. Verbindungen gemäss Anspruch 1 der Formel I, worin R₁ und R₂ unabhängig voneinander Wasserstoff, C₁₋₇-Alkyl, C₁₋₇-Alkoxy Halogen oder Trifluormethyl bedeuten, R₃ Wasserstoff oder C₁₋₇-Alkyl bedeutet, der durch die Formel 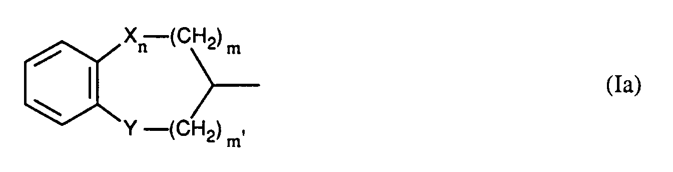 dargestellte Benzoheterocyclylrest 2,3-Dihydrobenzofuranyl, Chromanyl oder 1,4-Benzodioxanyl darstellt und alk₁ und alk₂ gleiche oder verschiedene C₁₋₇-Alkylenreste bedeuten, und ihre Salze.

4. Verbindungen gemäss Anspruch 2 der Formel I, worin R₁ und R₂ unabhängig voneinander Wasserstoff, C₁-C₄-Alkyl, C₁-C₄-Alkoxy oder Halogen der Atomnummer bis und mit 35 bedeuten, R₃ Wasserstoff oder C₁-C₇-Alkyl bedeutet, der durch die Formel Ia dargestellte Benzoheterocyclylrest 2,3-Dihydrobenzofuranyl, 2,3-Dihydrobenzothienyl, Chromanyl, Thiochromanyl, 1,4-Benzodioxanyl, 1,4-Benzoxathianyl, 1,4-Benzodithianyl, 2,3,4,5-Tetrahydrobenzoxepinyl, 2,3,4,5-Tetrahydrobenzothiepinyl, 2,3,4,5-Tetrahydrobenzodioxepinyl, 2,3,4,5-Tetrahydrobenzoxathiepinyl oder 2,3,4,5-Tetrahydrobenzodithiepinyl darstellt und alk₁ und alk₂ gleiche oder verschiedene C₁-C₄-Alkylenreste bedeuten, und ihre Salze.

5. Verbindungen gemäss Anspruch 2 der Formel I, worin R₁ und R₂ unabhängig voneinander Wasserstoff, C₁-C₄-Alkyl, C₁-C₄-Alkoxy oder Halogen der Atomnummer bis und mit 35 bedeuten, R₃ Wasserstoff oder C₁-C₄-Alkyl bedeutet, der durch die Formel Ia dargestellte Benzoheterocyclylrest 2,3-Dihydrobenzofuranyl, Chromanyl oder 1,4-Benzodioxanyl darstellt, alk₁ C₁-C₄-Alkylen bedeutet und und alk₂ C₂-C₃-Alkylen bedeutet, und ihre Salze.

6. Verbindungen gemäss Anspruch 2 der Formel I, worin R₁ Wasserstoff, C₁-C₄-Alkyl, C₁-C₄-Alkoxy oder Halogen der Atomnummer bis und mit 35 bedeutet, R₂ Wasserstoff darstellt, R₃ Wasserstoff oder C₁-C₄-Alkyl bedeutet, der durch die Formel Ia dargestellte Benzoheterocyclylrest 2,3-Dihydrobenzofuranyl, Chromanyl oder 1,4-Benzodioxanyl bedeutet, alk₁ C₁-C₃-Alkylen bedeutet und alk₂ Äthylen darstellt, und ihre Salze.

7. Verbindung gemäß Anspruch 1, worin die Verbindung 3-{N-[2-{Chroman-3-yl)äthyl]amino}-1-hydroxy-propan-1,1-diphosphonsäure oder ein Salz davon ist.

8. Verbindung gemäß Anspruch 1, worin die Verbindung 3-{N-[2-(Chroman-3-yl)äthyl]-N-methyl-amino}-1-hydroxy-propan-1,1-diphosphonsäure oder ein Salz davon ist.

9. Verbindung gemäß Anspruch 1, worin die Verbindung 3-[N-(6-Methyl-1,4-benzodioxan-2-yl)methyl-N-methyl-amino]-1-hydroxy-propan-1,1-diphosphonsäure oder ein Salz davon ist.

10. Verbindung gemäß Anspruch 1, worin die Verbindung 3-[N-(6-Methoxy-1,4-benzodioxan-2-yl)methyl-N-methyl-amino]-1-hydroxy-propan-1,1-diphosphonsäure oder ein Salz davon ist.

11. Verbindung gemäß Anspruch 1, worin die Verbindung 3-[N-(7-Chlor-1,4-benzodioxan-2-yl)methyl-N-methyl-amino]-1-hydroxy-propan-1,1-diphosphonsäure oder ein Salz davon ist.

12. Verbindung gemäß Anspruch 1, worin die Verbindung 3-[N-(2,3-Dihydrobenzothien-2-yl)methyl-N-methyl-amino]-1-hydroxy-propan-1,1-diphosphonsäure oder ein Salz davon ist.

13. Verbindung gemäß Anspruch 1, worin die Verbindung 3-{N-[2-(Chroman-2-yl)äthyl]-N-methyl-amino}-1-hydroxy-propan-1,1-diphosphonsäure oder ein Salz davon ist.

14. Verbindung gemäß Anspruch 1, worin die Verbindung 3-[N-(6-Methylbenzo-1,4-dioxan-2-ylmethyl)amino]-1-hydroxy-propan-1,1-diphosphonsäure oder ein Salz davon ist.

15. Verbindung gemäß Anspruch 1, worin die Verbindung 3-[N-(Benzo-1,4-dioxan-2-ylmethyl)-N-methyl-amino]-1-hydroxy-propan-1,1-diphosphonsäure oder ein Salz davon ist.

16. Verbindung gemäß Anspruch 1, worin die Verbindung 3-[N-(5-Methoxy-2,3-dihydro-benzofuran-2-yl)methylamino]-1-hydroxy-propan-1,1-diphosphonsäure oder ein Salz davon ist.

17. Verbindung gemäß Anspruch 1, worin die Verbindung 3-[N-(6-Chlorbenzo-1,4-dioxan-2-ylmethyl)-N-methyl-amino]-1-hydroxy-propan-1,1-diphosphonsäure oder ein Salz davon ist.

18. Verbindungen gemäss einem der Ansprüche 1 bis 17 zur Anwendung in einem Verfahren zur therapeutischen Behandlung des menschlichen oder tierischen Körpers.

19. Pharmazeutische Präparate, neben pharmazeutischen Hilfsstoffen als pharmazeutischen Wirkstoff enthaltend eine Verbindung gemäss einem der Ansprüche 1 bis 18 in freier Form oder in pharmazeutisch verwendbarer Salzform.

20. Verfahren zur Herstellung von Benzoheterocyclylalkylaminoalkandiphosphonsäuren der Formel I gemäss Anspruch 1 oder Salzen davon, dadurch gekennzeichnet, dass man
a) in einer Verbindung der Formel II 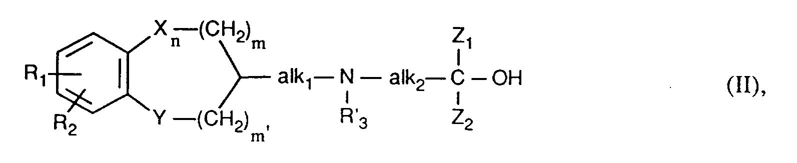 worin R₁, R₂, n, m, m', X, Y, alk₁ und alk₂ die angegebenen Bedeutungen haben, R'₃ eine der genannten Gruppen R₃ oder eine Aminoschutzgrüppe R₀, Z₁ eine funktionell abgewandelte und Z₂ eine freie oder funktionell abgewandelte Phosphonogruppe bedeutet, funktionell abgewandeltes Phosphono Z₁ und gegebenenfalls Z₂ in die freie Phosphonogruppe überführt oder
b) Verbindungen der Formeln IIIa und IVa 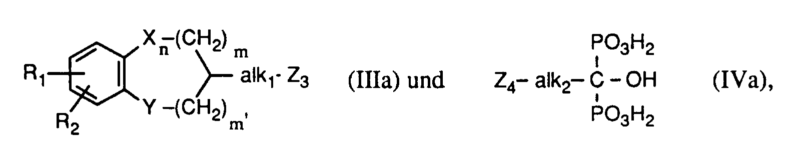 worin R₁, R₂, n, m, m', X, Y, alk₁ und alk₂ die angegebenen Bedeutungen haben, einer der Reste Z₃ und Z₄ eine reaktionsfähige veresterte Hydroxygruppe und der andere eine Gruppe der Formel -N(R'₃)-H darstellt, in der R'₃ eine der genannten Gruppen R₃ oder eine Aminoschutzgruppe R₀ bedeutet, oder deren Salze oder unter reduzierenden Bedingungen Verbindungen der Formeln IIIb und IVb 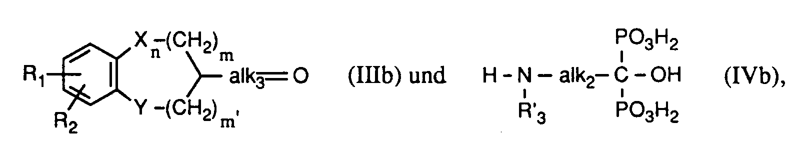 worin alk₃ einen dem Rest alk₁ entsprechenden doppelt gebundenen Rest, d.h. einen C₁₋₇-Alkanylylidenrest, darstellt, R'₃ eine der genannten Gruppen R₃ oder eine Aminoschutzgruppe R₀ bedeutet und R₁, R₂, n, m, m', X, Y, alk₁ und alk₂ die angegebenen Bedeutungen haben, miteinander umsetzt oder
c) eine Verbindung der Formel V 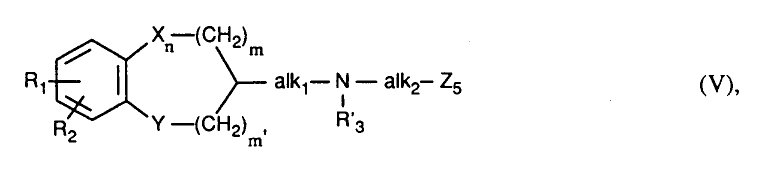 worin Z₅ Carboxy, Carbamoyl oder Cyano und R'₃ eine der genannten Gruppen R₃ oder eine Aminoschutzgruppe R₀ bedeutet und R₁, R₂, n, m, m', X, Y, alk₁ und alk₂ die angegebenen Bedeutungen haben, mit einem Phosphorylierungsmittel umsetzt, das Primärprodukt hydrolysiert und in einem ausgehend von Verbindungen der Formel V, worin Z₅ Cyano oder Carbamoyl ist, erhaltenen Zwischenprodukt der Formel Va 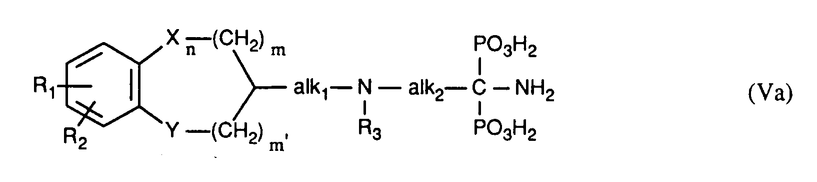 bzw. einem Salz davon die Aminogruppe durch Behandlung mit salpetriger Säure durch Hydroxy ersetzt, jeweils die Aminoschutzgruppe R₀, sofern vorhanden abspaltet und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

21. Verwendung einer Verbindung gemäss einem der Ansprüche 1 bis 18 zur Herstellung eines für die Behandlung von Erkrankungen, die mit Störungen des Calcium-Stoffwechsels in Verbindung gebracht werden können, von entzündlichen Prozessen in Gelenken, degenerativen Prozessen im Gelenkknorpel, ferner von Calciumablagerungen in Blutgefässen oder in prothetischen Implantaten, insbesondere von tumorinduzierten Hyperkalzämien, von Knochenmatastasen und des morbus Paget, von Osteoporosis, Periodontitis und Hyperparathyreoidismus, bestimmten Arzneimittels.

## Patentansprüche (Patentansprüche für folgende(n) Vertragsstaat(en): ES, GR)

1. Verfahren zur Herstellung von Benzoheterocyclylalkylaminoalkandiphosphonsäuren der Formel I 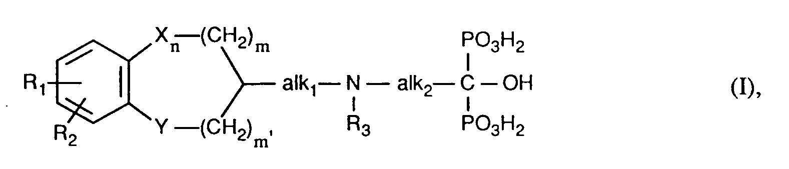 worin R₁ und R₂ unabhängig voneinander Wasserstoff, C₁-C₇-Alkyl, C₁-C₇-Alkoxy, Halogen oder Trifluormethyl bedeuten, R₃ Wasserstoff oder C₁-C₇-Alkyl bedeutet, X und Y unabhängig voneinander Oxy oder Thio darstellen, alk₁ und alk₂ gleiche oder verschiedene C₁-C₇-Alkylenreste bedeuten, n für 0 oder 1 steht und m und m' unabhängig voneinander für 0, 1 oder 2 stehen, wobei die Summe von n, m und m' 1, 2 oder 3 ist, und ihrer Salze, dadurch gekennzeichnet, dass man
a) in einer Verbindung der Formel II 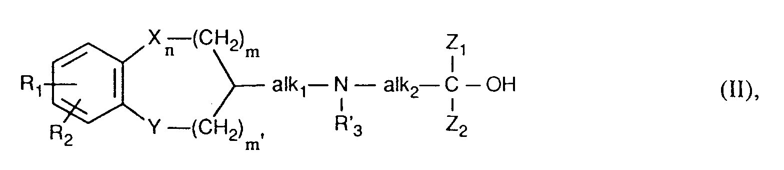 worin R₁, R₂, n, m, m', X, Y, alk₁ und alk₂ die angegebenen Bedeutungen haben, R'₃ eine der genannten Gruppen R₃ oder eine Aminoschutzgrüppe R₀, Z₁ eine funktionell abgewandelte und Z₂ eine freie oder funktionell abgewandelte Phosphonogruppe bedeutet, funktionell abgewandeltes Phosphono Z₁ und gegebenenfalls Z₂ in die freie Phosphonogruppe überführt oder
b) Verbindungen der Formeln IIIa und IVa 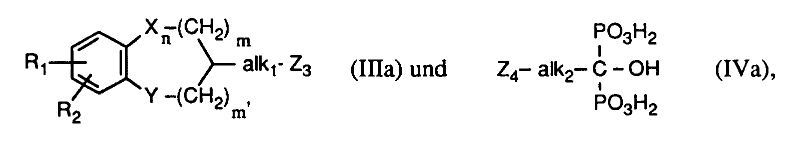 worin R₁, R₂, n, m, m', X, Y, alk₁ und alk₂ die angegebenen Bedeutungen haben, einer der Reste Z₃ und Z₄ eine reaktionsfähige veresterte Hydroxygruppe und der andere eine Gruppe der Formel -N(R'₃)-H darstellt, in der R'₃ eine der genannten Gruppen R₃ oder eine Aminoschutzgruppe R₀ bedeutet, oder deren Salze oder unter reduzierenden Bedingungen Verbindungen der Formeln IIIb und IVb 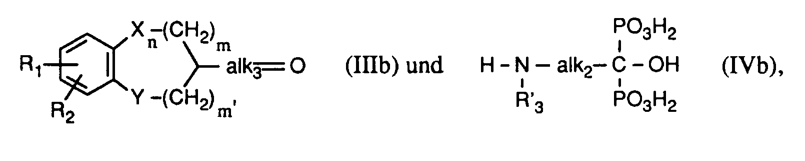 worin alk₃ einen dem Rest alk₁ entsprechenden doppelt gebundenen Rest, d.h. einen C₁₋₇-Alkanylylidenrest, darstellt, R'₃ eine der genannten Gruppen R₃ oder eine Aminoschutzgruppe R₀ bedeutet und R₁, R₂, n, m, m', X, Y, alk₁ und alk₂ die angegebenen Bedeutungen haben, miteinander umsetzt oder
c) eine Verbindung der Formel V 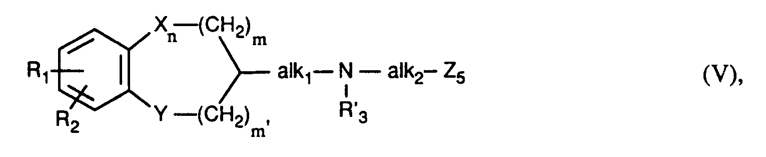 worin Z₅ Carboxy, Carbamoyl oder Cyano und R'₃ eine der genannten Gruppen R₃ oder eine Aminoschutzgruppe R₀ bedeutet und R₁, R₂, n, m, m', X, Y, alk₁ und alk₂ die angegebenen Bedeutungen haben, mit einem Phosphorylierungsmittel umsetzt, das Primärprodukt hydrolysiert und in einem ausgehend von Verbindungen der Formel V, worin Z₅ Cyano oder Carbamoyl ist, erhaltenen Zwischenprodukt der Formel Va 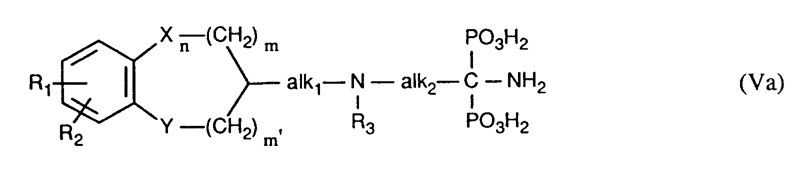 bzw. einem Salz davon die Aminogruppe durch Behandlung mit salpetriger Säure durch Hydroxy ersetzt, jeweils die Aminoschutzgrüppe R₀, sofern vorhanden abspaltet und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

2. Verfahren gemäss Anspruch 1, dadurch gekennzeichnet, dass man eine Verbindung der Formel I, worin R₁ und R₂ unabhängig voneinander Wasserstoff, C₁-C₇-Alkyl, C₁-C₇-Alkoxy, Halogen oder Trifluormethyl bedeuten, R₃ Wasserstoff oder C₁-C₇-Alkyl bedeutet, der durch die Formel 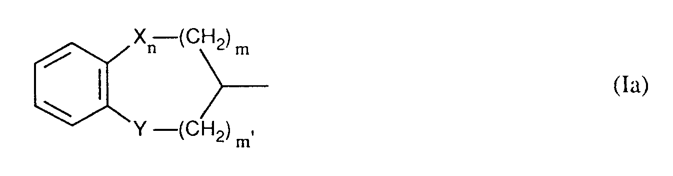 dargestellte Benzoheterocyclylrest 2,3-Dihydrobenzofuranyl, 2,3-Dihydrobenzothienyl, Chromanyl, Thiochromanyl, 1,4-Benzodioxanyl, 1,4-Benzoxathianyl, 1,4-Benzodithianyl 2,3,4,5-Tetrahydrobenzoxepinyl, 2,3,4,5-Tetrahydrobenzothiepinyl, 2,3,4,5-Tetrahydrobenzodioxepinyl, 2,3,4,5-Tetrahydrobenzoxathiepinyl oder 2,3,4,5-Tetrahydrobenzodithiepinyl darstellt und alk₁ und alk₂ gleiche oder verschiedene C₁-C₇-Alkylenreste bedeuten, oder ein Salz davon herstellt, wobei die genannten Benzoheterocyclylreste unsubstituiert oder durch einen von Wasserstoff verschiedenen Rest R₁ mono- oder durch gleiche oder verschiedene Reste R₁ und R₂ disubstituiert sind.

3. Verfahren gemäss Anspruch 1, dadurch gekennzeichnet, dass man eine Verbindung der Formel I worin R₁ und R₂ unabhängig voneinander Wasserstoff, C₁-C₇-Alkyl, C₁-C₇-Alkoxy, Halogen oder Trifluormethyl bedeuten, R₃ Wasserstoff oder C₁-C₇-Alkyl bedeutet, der durch die Formel 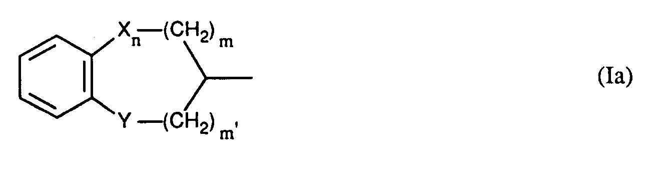 dargestellte Benzoheterocyclylrest 2,3-Dihydrobenzofuranyl, Chromanyl oder 1,4-Benzodioxanyl darstellt und alk₁ und alk₂ gleiche oder verschiedene Niederalkylenreste bedeuten, oder ein Salz davon herstellt.

4. Verfahren gemäss Anspruch 2, dadurch gekennzeichnet, dass man eine Verbindung der Formel I, worin R₁ und R₂ unabhängig voneinander Wasserstoff, C₁-C₄-Alkyl, C₁-C₄-Alkoxy oder Halogen der Atomnummer bis und mit 35 bedeuten, R₃ Wasserstoff oder C₁-C₇-Alkyl bedeutet, der durch die Formel Ia dargestellte Benzoheterocyclylrest 2,3-Dihydrobenzofuranyl, 2,3-Dihydrobenzothienyl, Chromanyl, Thiochromanyl, 1,4-Benzodioxanyl, 1,4-Benzoxathianyl, 1,4-Benzodithianyl, 2,3,4,5-Tetrahydrobenzoxepinyl, 2,3,4,5-Tetrahydrobenzothiepinyl, 2,3,4,5-Tetrahydrobenzodioxepinyl, 2,3,4,5-Tetrahydrobenzoxathiepinyl oder 2,3,4,5-Tetrahydrobenzodithiepinyl darstellt und alk₁ und alk₂ gleiche oder verschiedene C₁-C₄-Alkylenreste bedeuten, oder ein Salz davon herstellt.

5. Verfahren gemäss Anspruch 2, dadurch gekennzeichnet, dass man eine Verbindung der Formel I, worin R₁ und R₂ unabhängig voneinander Wasserstoff, C₁-C₄-Alkyl, C₁-C₄-Alkoxy oder Halogen der Atomnummer bis und mit 35 bedeuten, R₃ Wasserstoff oder C₁-C₄-Alkyl bedeutet, der durch die Formel Ia dargestellte Benzoheterocyclylrest 2,3-Dihydrobenzofuranyl, Chromanyl oder 1,4-Benzodioxanyl darstellt, alk₁ C₁-C₄-Alkylen bedeutet und und alk₂ C₂-C₃-Alkylen bedeutet, oder ein Salz davon herstellt.

6. Verfahren gemäss Anspruch 2, dadurch gekennzeichnet, dass man eine Verbindung der Formel I, worin R₁ Wasserstoff, C₁-C₄-Alkyl, C₁-C₄-Alkoxy oder Halogen der Atomnummer bis und mit 35 bedeutet, R₂ Wasserstoff darstellt, R₃ Wasserstoff oder C₁-C₄-Alkyl bedeutet, der durch die Formel Ia dargestellte Benzoheterocyclylrest 2,3-Dihydrobenzofuranyl, Chromanyl oder 1,4-Benzodioxanyl bedeutet, alk₁ C₁-C₃-Alkylen, wie Methylen, Äthylen oder 1,3-Propylen, bedeutet und alk₂ Äthylen darstellt, oder ein Salz davon herstellt.

7. Verfahren gemäss Anspruch 2, dadurch gekennzeichnet, dass man 3-{N-[2-{Chroman-3-yl)äthyl]amino}-1-hydroxy-propan-1,1-diphosphonsäure oder ein Salz davon herstellt.

8. Verfahren gemäss Anspruch 2, dadurch gekennzeichnet, dass man 3-{N-[2-(Chroman-3-yl)äthyl)-N-methyl-amino}-1-hydroxy-propan-1,1-diphosphonsäure oder ein Salz davon herstellt.

9. Verfahren gemäss Anspruch 2, dadurch gekennzeichnet, dass man 3-[N-(6-Methyl-1,4-benzodioxan-2-yl)methyl-N-methyl-amino]-1-hydroxy-propan-1,1-diphosphonsäure oder ein Salz davon herstellt.

10. Verfahren gemäss Anspruch 2, dadurch gekennzeichnet, dass man 3-[N-(6-Methoxy-1,4-benzodioxan-2-yl)methyl-N-methyl-amino]-1-hydroxy-propan-1,1-diphosphonsäure oder ein Salz davon herstellt.

11. Verfahren gemäss Anspruch 2, dadurch gekennzeichnet, dass man 3-[N-(7-Chlor-1,4-benzodioxan-2-yl)methyl-N-methyl-amino]-1-hydroxy-propan-1,1-diphosphonsäure oder ein Salz davon herstellt.

12. Verfahren gemäss Anspruch 1, dadurch gekennzeichnet, dass man 3-[N-(2,3-Dihydrobenzothien-2-yl)methyl-N-methyl-amino]-1-hydroxy-propan-1,1-diphosphonsäure oder ein Salz davon herstellt.

13. Verfahren gemäss Anspruch 1, dadurch gekennzeichnet, dass man 3-{N-[2-(Chroman-2-yl)äthyl]-N-methyl-amino]-1-hydroxy-propan-1,1-diphosphonsaure oder ein Salz davon herstellt.

14. Verfahren gemäss Anspruch 1, dadurch gekennzeichnet, dass man 3-[N-(6-Methylbenzo-1,4-dioxan-2-ylmethyl)amino]-1-hydroxy-propan-1,1-diphosphonsäure oder ein Salz davon herstellt.

15. Verfahren gemäss Anspruch 1, dadurch gekennzeichnet, dass man 3-[N-(Benzo-1,4-dioxan-2-ylmethyl)-N-methyl-amino]-1-hydroxy-propan-1,1-diphosphonsäure oder ein Salz davon herstellt.

16. Verfahren gemäss Anspruch 1, dadurch gekennzeichnet, dass man 3-[N-(5-Methoxy-2,3-dihydro-benzofuran-2-yl)methylamino]-1-hydroxy-propan-1,1-diphosphonsäure oder ein Salz davon herstellt.

17. Verfahren gemäss Anspruch 1, dadurch gekennzeichnet, dass man 3-[N-(6-Chlorbenzo-1,4-dioxan-2-ylmethyl)-N-methyl-amino]-1-hydroxy-propan-1,1-diphosphonsäure oder ein Salz davon herstellt.

18. Verfahren zur Herstellung von für die Behandlung von Störungen des Calciumstoffwechsels geeigneten pharmazeutischen Präparaten, dadurch gekennzeichnet, dass man eine gemäss einem der Ansprüche 1 bis 17 erhältliche Verbindung in an sich bekannter Weise, allein oder mit für die Herstellung von Arzneimittels gebräuchlichen pharmazeutischen Hilfsstoffen, gegebenenfalls in Kombination mit anderen Arzneimittelwirkstoffen, in die Form von Tabletten, Kapseln, Pillen, Suppositorien, Emulsionen, Injektions- oder Infusionslösungen oder andere bekannte Arzneimittelformen bringt.

## Claims (Claims for the following Contracting State(s): AT, BE, CH, DE, DK, FR, GB, IT, LI, LU, NL, SE)

1. A benzoheterocyclylalkylaminoalkanediphosphonic acid of formula I 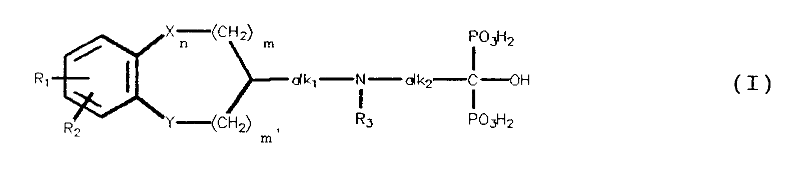 wherein R₁ and R₂, independently of one another, are hydrogen, C₁-C₇alkyl, C₁-C₇alkoxy, halogen or trifluoromethyl, R₃ is hydrogen or C₁-C₇alkyl, X and Y, independently of one another, are oxy or thio, alk₁ and alk₂ are identical or different C₁-C₇alkylene radicals, n is 0 or 1 and m and m', independently of one another, are 0, 1 or 2, the sum of n, m and m' being 1, 2 or 3, or a salt thereof.

2. A compound according to claim 1 of formula I wherein R₁ and R₂, independently of one another, are hydrogen, C₁-C₇alkyl, C₁-C₇alkoxy, halogen or trifluoromethyl, R₃ is hydrogen or C₁-C₇alkyl, the benzoheterocyclyl radical of formula 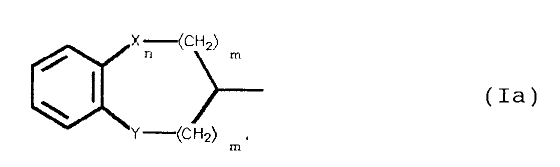 is 2,3-dihydrobenzofuranyl, 2,3-dihydrobenzothienyl, chromanyl, thiochromanyl, 1,4-benzodioxanyl, 1,4-benzoxathianyl, 1,4-benzodithianyl, 2,3,4,5-tetrahydrobenzoxepinyl, 2,3,4,5-tetrahydrobenzothiepinyl, 2,3,4,5-tetrahydrobenzodioxepinyl, 2,3,4,5-tetrahydrobenzoxathiepinyl or 2,3,4,5-tetrahydrobenzodithiepinyl and alk₁ and alk₂ are identical or different C₁-C₇alkylene radicals, or a salt thereof, wherein the said benzoheterocyclyl radicals are unsubstituted, are monosubstituted by a radical R₁ other than hydrogen or are disubstituted by identical or different radicals R₁ and R₂.

3. A compound according to claim 1 of formula I wherein R₁ and R₂, independently of one another, are hydrogen, C₁-C₇alkyl, C₁-C₇alkoxy, halogen or trifluoromethyl, R₃ is hydrogen or C₁-C₇alkyl, the benzoheterocyclyl radical of formula 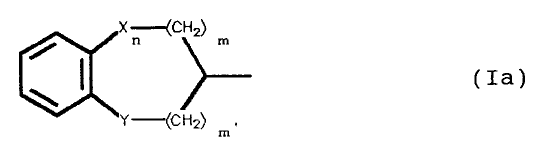 is 2,3-dihydrobenzofuranyl, chromanyl or 1,4-benzodioxanyl and alk₁ and alk₂ are identical or different C₁-C₇alkylene radicals, or a salt thereof.

4. A compound according to claim 2 of formula I wherein R₁ and R₂, independently of one another, are hydrogen, C₁-C₄alkyl, C₁-C₄alkoxy or halogen having an atomic number of up to and including 35, R₃ is hydrogen or C₁-C₇alkyl, the benzoheterocyclyl radical of formula Ia is 2,3-dihydrobenzofuranyl, 2,3-dihydrobenzothienyl, chromanyl, thiochromanyl, 1,4-benzodioxanyl, 1,4-benzoxathianyl, 1,4-benzodithianyl, 2,3,4,5-tetrahydrobenzoxepinyl, 2,3,4,5-tetrahydrobenzothiepinyl, 2,3,4,5-tetrahydrobenzodioxepinyl, 2,3,4,5-tetrahydrobenzoxathiepinyl or 2,3,4,5-tetrahydrobenzodithiepinyl and alk₁ and alk₂ are identical or different C₁-C₄alkylene radicals, or a salt thereof.

5. A compound according to claim 2 of formula I wherein R₁ and R₂, independently of one another, are hydrogen, C₁-C₄alkyl, C₁-C₄alkoxy or halogen having an atomic number of up to and including 35, R₃ is hydrogen or C₁-C₄alkyl, the benzoheterocyclyl radical of formula Ia is 2,3-dihydrobenzofuranyl, chromanyl or 1,4-benzodioxanyl, alk₁ is C₁-C₄alkylene and alk₂ is C₂-C₃alkylene, or a salt thereof.

6. A compound according to claim 2 of formula I wherein R₁ is hydrogen, C₁-C₄alkyl, C₁-C₄alkoxy or halogen having an atomic number of up to and including 35, R₂ is hydrogen, R₃ is hydrogen or C₁-C₄alkyl, the benzoheterocyclyl radical of formula Ia is 2,3-dihydrobenzofuranyl, chromanyl or 1,4-benzodioxanyl, alk₁ is C₁-C₃alkylene and alk₂ is ethylene, or a salt thereof.

7. A compound according to claim 1, wherein the compound is 3-{N-[2-(chroman-3-yl)ethyl]amino}-1-hydroxypropane-1,1-diphosphonic acid or a salt thereof.

8. A compound according to claim 1, wherein the compound is 3-{N-[2-(chroman-3-yl)ethyl]-N-methylamino}-1-hydroxypropane-1,1-diphosphonic acid or a salt thereof.

9. A compound according to claim 1, wherein the compound is 3-[N-(6-methyl-1,4-benzodioxan-2-yl)methyl-N-methylamino]-1-hydroxypropane-1,1-diphosphonic acid or a salt thereof.

10. A compound according to claim 1, wherein the compound is 3-[N-(6-methoxy-1,4-benzodioxan-2-yl)methyl-N-methylamino]-1-hydroxypropane-1,1-diphosphonic acid or a salt thereof.

11. A compound according to claim 1, wherein the compound is 3-[N-(7-chloro-1,4-benzodioxan-2-yl)methyl-N-methylamino]-1-hydroxypropane-1,1-diphosphonic acid or a salt thereof.

12. A compound according to claim 1, wherein the compound is 3-[N-(2,3-dihydrobenzothien-2-yl)methyl-N-methylamino]-1-hydroxypropane-1,1-diphosphonic acid or a salt thereof.

13. A compound according to claim 1, wherein the compound is 3-{N-[2-(chroman-2-yl)ethyl]-N-methylamino}-1-hydroxypropane-1,1-diphosphonic acid or a salt thereof.

14. A compound according to claim 1, wherein the compound is 3-[N-(6-methylbenzo-1,4-dioxan-2-ylmethyl)amino]-1-hydroxypropane-1,1-diphosphonic acid or a salt thereof.

15. A compound according to claim 1, wherein the compound is 3-[N-(benzo-1,4-dioxan-2-ylmethyl)-N-methylamino]-1-hydroxypropane-1,1-diphosphonic acid or a salt thereof.

16. A compound according to claim 1, wherein the compound is 3-[N-(5-methoxy-2,3-dihydrobenzofuran-2-yl)methylamino]-1-hydroxypropane-1,1-diphosphonic acid or a salt thereof.

17. A compound according to claim 1, wherein the compound is 3-[N-(6-chlorobenzo-1,4-dioxan-2-ylmethyl)-N-methylamino]-1-hydroxypropane-1,1-diphosphonic acid or a salt thereof.

18. A compound according to any one of claims 1 to 17 for use in a method for the therapeutic treatment of the human or animal body.

19. A pharmaceutical composition comprising as pharmaceutical active ingredient a compound according to any one of claims 1 to 18 in free form or in the form of a pharmaceutically acceptable salt, together with pharmaceutical excipients.

20. A process for the preparation of a benzoheterocyclylalkylaminoalkanediphosphonic acid of formula I according to claim 1 or a salt thereof, which process comprises
a) in a compound of formula II 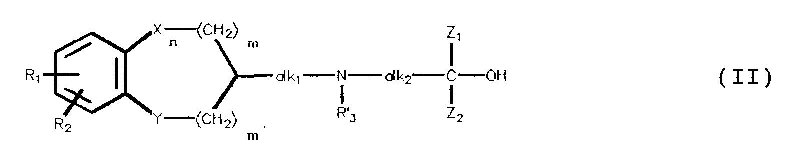 wherein R₁, R₂, n, m, m', X, Y, alk₁ and alk₂ are as defined above, R'₃ is one of the groups R₃ mentioned above or is an amino-protecting group R₀, Z₁ is a functionally modified phosphono group and Z₂ is a free or functionally modified phosphono group, converting functionally modified phosphono Z₁ and, where appropriate, Z₂ into the free phosphono group, or
b) reacting with one another compounds of formulae IIIa and IVa 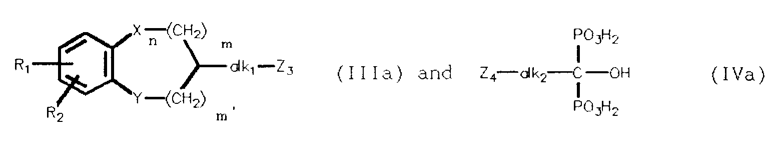 wherein R₁, R₂, n, m, m', X, Y, alk₁ and alk₂ are as defined above, one of the radicals Z₃ and Z₄ is a reactive esterified hydroxy group and the other is a group of the formula -N(R'₃)-H wherein R'₃ is one of the groups R₃ mentioned above or is an amino-protecting group R₀, or salts thereof, or, under reducing conditions, reacting with one another compounds of formulae IIIb and IVb 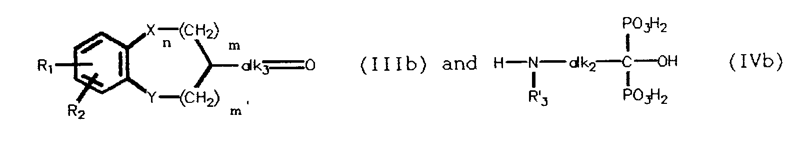 wherein alk₃ is a double-bonded radical corresponding to the radical alk₁, that is to say, a C₁-C₇alkanylylidene radical, R'₃ is one of the groups R₃ mentioned above or is an amino-protecting group R₀, and R₁, R₂, n, m, m', X, Y, alk₁ and alk₂ are as defined above, or
c) reacting a compound of formula V 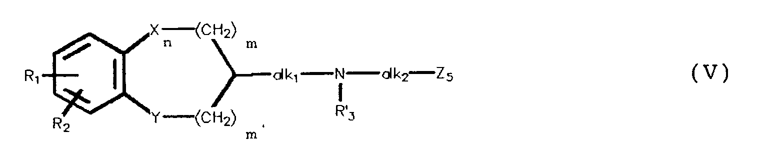 wherein Z₅ is carboxy, carbamoyl or cyano and R'₃ is one of the groups R₃ mentioned above or is an amino-protecting group R₀, and R₁, R₂, n, m, m', X, Y, alk₁ and alk₂ are as defined above, with a phosphorylating agent, hydrolysing the primary product and, in an intermediate of formula Va 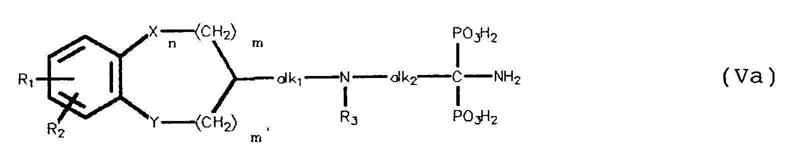 obtained starting from a compound of formula V wherein Z₅ is cyano or carbamoyl, or in a salt thereof, replacing the amino group with hydroxy by treatment with nitrous acid, in each case removing the amino-protecting group R₀, if present, and, if desired, converting a resulting compound into a different compound of formula I, separating a mixture of isomers obtainable in accordance with the process into the components and separating out the preferred isomer, and/or converting a free compound obtainable in accordance with the process into a salt or converting a salt obtainable in accordance with the process into the corresponding free compound.

21. The use of a compound according to any one of claims 1 to 18 for the preparation of a medicament for the treatment of diseases that may be associated with calcium metabolism disorders, of inflammatory processes in joints, of degenerative processes in the articular cartilage, and of calcium deposits in blood vessels or in prosthetic implants, especially tumour-induced hypercalcaemia, bone metastases and Paget's disease, osteoporosis, periodontitis and hyperparathyroidism.

## Claims (Claims for the following Contracting State(s): ES, GR)

1. A process for the preparation of a benzoheterocyclylalkylaminoalkanediphosphonic acid of formula I 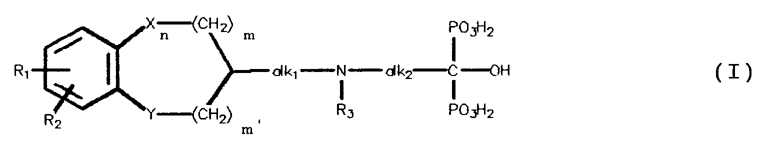 wherein R₁ and R₂, independently of one another, are hydrogen, C₁-C₇alkyl, C₁-C₇alkoxy, halogen or trifluoromethyl, R₃ is hydrogen or C₁-C₇alkyl, X and Y, independently of one another, are oxy or thio, alk₁ and alk₂ are identical or different C₁-C₇alkylene radicals, n is 0 or 1 and m and m', independently of one another, are 0, 1 or 2, the sum of n, m and m' being 1, 2 or 3, or a salt thereof, which process comprises
a) in a compound of formula II 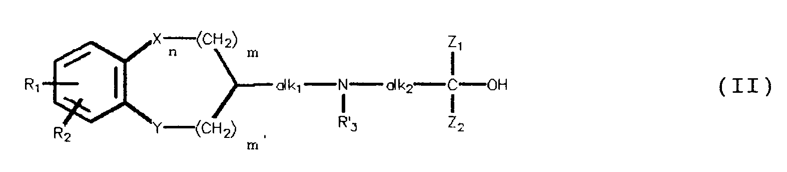 wherein R₁, R₂, n, m, m', X, Y, alk₁ and alk₂ are as defined above, R'₃ is one of the groups R₃ mentioned above or is an amino-protecting group R₀, Z₁ is a functionally modified phosphono group and Z₂ is a free or functionally modified phosphono group, converting functionally modified phosphono Z₁ and, where appropriate, Z₂ into the free phosphono group, or
b) reacting with one another compounds of formulae IIIa and IVa 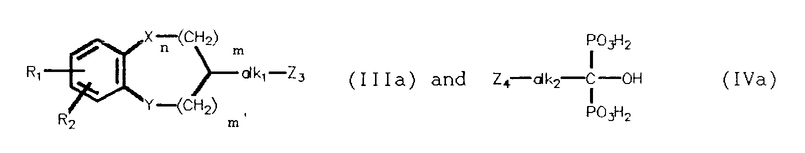 wherein R₁, R₂, n, m, m', X, Y, alk₁ and alk₂ are as defined above, one of the radicals Z₃ and Z₄ is a reactive esterified hydroxy group and the other is a group of the formula -N(R'₃)-H wherein R'₃ is one of the groups R₃ mentioned above or is an amino-protecting group R₀, or salts thereof, or, under reducing conditions, reacting with one another compounds of formulae IIIb and IVb 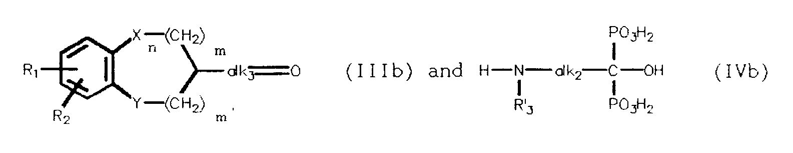 wherein alk₃ is a double-bonded radical corresponding to the radical alk₁, that is to say, a C₁-C₇alkanylylidene radical, R'₃ is one of the groups R₃ mentioned above or is an amino-protecting group R₀, and R₁, R₂, n, m, m', X, Y, alk₁ and alk₂ are as defined above, or
c) reacting a compound of formula V 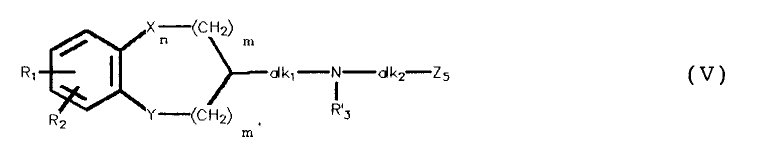 wherein Z₅ is carboxy, carbamoyl or cyano and R'₃ is one of the groups R₃ mentioned above or is an amino-protecting group R₀, and R₁, R₂, n, m, m', X, Y, alk₁ and alk₂ are as defined above, with a phosphorylating agent, hydrolysing the primary product and, in an intermediate of formula Va 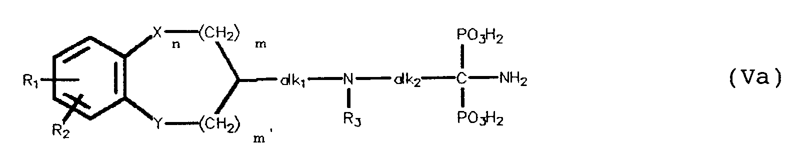 obtained starting from a compound of formula V wherein Z₅ is cyano or carbamoyl, or in a salt thereof, replacing the amino group with hydroxy by treatment with nitrous acid, in each case removing the amino-protecting group R₀, if present, and, if desired, converting a resulting compound into a different compound of formula I, separating a mixture of isomers obtainable in accordance with the process into the components and separating out the preferred isomer, and/or converting a free compound obtainable in accordance with the process into a salt or converting a salt obtainable in accordance with the process into the corresponding free compound.

2. A process according to claim 1, which comprises preparing a compound of formula I wherein R₁ and R₂, independently of one another, are hydrogen, C₁-C₇alkyl, C₁-C₇alkoxy, halogen or trifluoromethyl, R₃ is hydrogen or C₁-C₇alkyl, the benzoheterocyclyl radical of formula 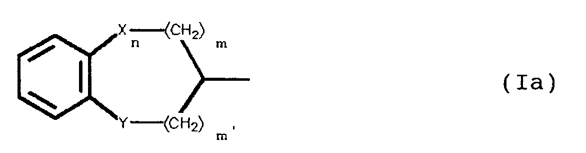 is 2,3-dihydrobenzofuranyl, 2,3-dihydrobenzothienyl, chromanyl, thiochromanyl, 1,4-benzodioxanyl, 1,4-benzoxathianyl, 1,4-benzodithianyl, 2,3,4,5-tetrahydrobenzoxepinyl, 2,3,4,5-tetrahydrobenzothiepinyl, 2,3,4,5-tetrahydrobenzodioxepinyl, 2,3,4,5-tetrahydrobenzoxathiepinyl or 2,3,4,5-tetrahydrobenzodithiepinyl and alk₁ and alk₂ are identical or different C₁-C₇alkylene radicals, or a salt thereof, wherein the said benzoheterocyclyl radicals are unsubstituted, are monosubstituted by a radical R₁ other than hydrogen or are disubstituted by identical or different radicals R₁ and R₂.

3. A process according to claim 1, which comprises preparing a compound of formula I wherein R₁ and R₂, independently of one another, are hydrogen, C₁-C₇alkyl, C₁-C₇alkoxy, halogen or trifluoromethyl, R₃ is hydrogen or C₁-C₇alkyl, the benzoheterocyclyl radical of formula 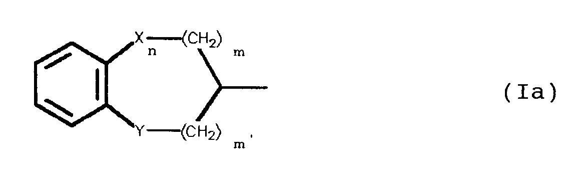 is 2,3-dihydrobenzofuranyl, chromanyl or 1,4-benzodioxanyl and alk₁ and alk₂ are identical or different lower alkylene radicals, or a salt thereof.

4. A process according to claim 2, which comprises preparing a compound of formula I wherein R₁ and R₂, independently of one another, are hydrogen, C₁-C₄alkyl, C₁-C₄alkoxy or halogen having an atomic number of up to and including 35, R₃ is hydrogen or C₁-C₇alkyl, the benzoheterocyclyl radical of formula Ia is 2,3-dihydrobenzofuranyl, 2,3-dihydrobenzothienyl, chromanyl, thiochromanyl, 1,4-benzodioxanyl, 1,4-benzoxathianyl, 1,4-benzodithianyl, 2,3,4,5-tetrahydrobenzoxepinyl, 2,3,4,5-tetrahydrobenzothiepinyl, 2,3,4,5-tetrahydrobenzodioxepinyl, 2,3,4,5-tetrahydrobenzoxathiepinyl or 2,3,4,5-tetrahydrobenzodithiepinyl and alk₁ and alk₂ are identical or different C₁-C₄alkylene radicals, or a salt thereof.

5. A process according to claim 2, which comprises preparing a compound of formula I wherein R₁ and R₂, independently of one another, are hydrogen, C₁-C₄alkyl, C₁-C₄alkoxy or halogen having an atomic number of up to and including 35, R₃ is hydrogen or C₁-C₄alkyl, the benzoheterocyclyl radical of formula Ia is 2,3-dihydrobenzofuranyl, chromanyl or 1,4-benzodioxanyl, alk₁ is C₁-C₄alkylene and alk₂ is C₂-C₃alkylene, or a salt thereof.

6. A process according to claim 2, which comprises preparing a compound of formula I wherein R₁ is hydrogen, C₁-C₄alkyl, C₁-C₄alkoxy or halogen having an atomic number of up to and including 35, R₂ is hydrogen, R₃ is hydrogen or C₁-C₄alkyl, the benzoheterocyclyl radical of formula Ia is 2,3-dihydrobenzofuranyl, chromanyl or 1,4-benzodioxanyl, alk₁ is C₁-C₃alkylene, such as methylene, ethylene or 1,3-propylene, and alk₂ is ethylene, or a salt thereof.

7. A process according to claim 2, which comprises preparing 3-{N-[2-(chroman-3-yl)ethyl]amino}-1-hydroxypropane-1,1-diphosphonic acid or a salt thereof.

8. A process according to claim 2, which comprises preparing 3-{N-[2-(chroman-3-yl)ethyl]-N-methylamino}-1-hydroxypropane-1,1-diphosphonic acid or a salt thereof.

9. A process according to claim 2, which comprises preparing 3-[N-(6-methyl-1,4-benzodioxan-2-yl)methyl-N-methylamino]-1-hydroxypropane-1,1-diphosphonic acid or a salt thereof.

10. A process according to claim 2, which comprises preparing 3-[N-(6-methoxy-1,4-benzodioxan-2-yl)methyl-N-methylamino]-1-hydroxypropane-1,1-diphosphonic acid or a salt thereof.

11. A process according to claim 2, which comprises preparing 3-[N-(7-chloro-1,4-benzodioxan-2-yl)methyl-N-methylamino]-1-hydroxypropane-1,1-diphosphonic acid or a salt thereof.

12. A process according to claim 1, which comprises preparing 3-[N-(2,3-dihydrobenzothien-2-yl)methyl-N-methylamino]-1-hydroxypropane-1,1-diphosphonic acid or a salt thereof.

13. A process according to claim 1, which comprises preparing 3-{N-[2-(chroman-2-yl)ethyl]-N-methylamino}-1-hydroxypropane-1,1-diphosphonic acid or a salt thereof.

14. A process according to claim 1, which comprises preparing 3-[N-(6-methylbenzo-1,4-dioxan-2-ylmethyl)amino]-1-hydroxypropane-1,1-diphosphonic acid or a salt thereof.

15. A process according to claim 1, which comprises preparing 3-[N-(benzo-1,4-dioxan-2-ylmethyl)-N-methylamino]-1-hydroxypropane-1,1-diphosphonic acid or a salt thereof.

16. A process according to claim 1, which comprises preparing 3-[N-(5-methoxy-2,3-dihydrobenzofuran-2-yl)methylamino]-1-hydroxypropane-1,1-diphosphonic acid or a salt thereof.

17. A process according to claim 1, which comprises preparing 3-[N-(6-chlorobenzo-1,4-dioxan-2-ylmethyl)-N-methylamino]-1-hydroxypropane-1,1-diphosphonic acid or a salt thereof.

18. A process for the preparation of a pharmaceutical composition that is suitable for the treatment of calcium metabolism disorders, wherein a compound obtainable in accordance with any one of claims 1 to 17 is formulated in a manner known per se, alone or together with pharmaceutical excipients customary for the preparation of medicaments, optionally in combination with other medicament active ingredients, into tablets, capsules, pills, suppositories, emulsions, injection or infusion solutions or other known dosage forms.

## Revendications (Revendications pour l'(les) Etat(s) contractant(s) suivant(s): AT, BE, CH, DE, DK, FR, GB, IT, LI, LU, NL, SE)

1. Acides benzohétérocyclylalkylaminoalcanediphosphoniques de formule I 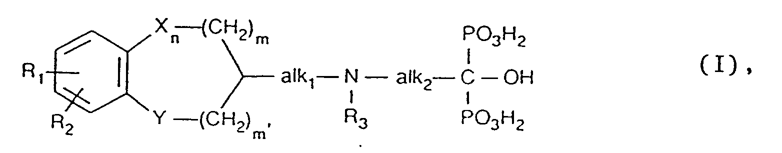 dans laquelle R₁ et R₂ représentent chacun, indépendamment l'un de l'autre, l'hydrogène, un groupe alkyle en C1-C7, alcoxy en C1-C7, un halogène ou un groupe trifluorométhyle, R₃ représente l'hydrogène ou un groupe alkyle en C1-C7, X et Y représentent chacun, indépendamment l'un de l'autre, un pont oxy ou thio, alk₁ et alk₂, ayant des significations identiques ou différentes, représentent chacun un groupe alkylène en C1-C7, n est égal à 0 ou 1 et m et m' sont égaux chacun, indépendamment l'un de l'autre, à 0, 1 ou 2, la somme de n, m et m' étant égale à 1, 2 ou 3, et leurs sels.

2. Composés selon revendication 1, de formule I dans laquelle R₁ et R₂ représentent chacun, indépendamment l'un de l'autre, l'hydrogène, un groupe alkyle en C1-C7, alcoxy en C1-C7, un halogène ou un groupe trifluorométhyle, R₃ représente l'hydrogène ou un groupe alkyle en C1-C7, le radical benzohétérocyclyle représenté par la formule 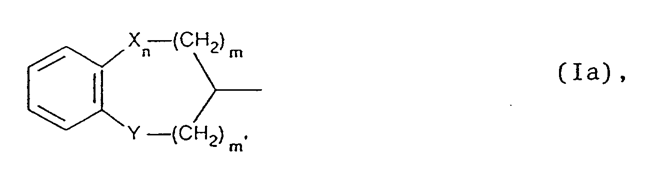 est un radical 2,3-dihydrobenzofurannyle, 2,3-dihydrobenzothiényle, chromannyle, thiochromannyle, 1,4-benzodioxannyle, 1,4-benzoxathiannyle, 1,4-benzodithiannyle, 2,3,4,5-tétrahydrobenzoxépinyie, 2,3,4,5-tétrahydrohenzothiépinyle, 2,3,4,5-tétrahydrohenzodioxépinyle, 2,3,4,5-tétrahydrobenzoxathiépinyle ou 2,3,4,5-tétrahydrobenzodithiépinyle, et alk₁ et alk₂, ayant des significations identiques ou différentes, représentent un groupe alkylène en C1-C7, et leurs sels, les radicaux benzohétérocyclyle mentionnés étant non substitués ou portant un substituant R₁ autre que l'hydrogène ou deux substituants R₁ et R₂ identiques ou différents.

3. Composés selon revendication 1 de formule I dans laquelle R₁ et R₂ représentent chacun, indépendamment l'un de l'autre, l'hydrogène, un groupe alkyle en C1-C7, alcoxy en C1-C7, un halogène ou un groupe trifluorométhyle, R₃ représente l'hydrogène ou un groupe alkyle en C1-C7, le radical benzohétérocyclyle représenté par la formule 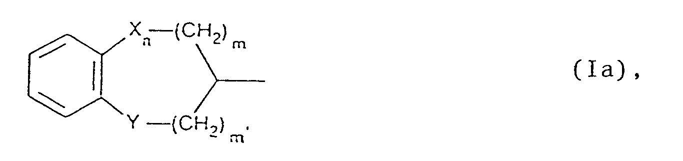 est un radical 2,3-dihydrobenzofurannyle, chromannyle ou 1,4-benzodioxannyle, et alk₁ et alk₂, ayant des significations identiques ou différentes, représentent chacun un groupe alkylène en C1-C7, et leurs sels.

4. Composés selon revendication 2, répondant à la formule I dans laquelle R₁ et R₂ représentent chacun, indépendamment l'un de l'autre, l'hydrogène, un groupe alkyle en C1-C4, alcoxy en C1-C4 ou un halogène de numéro atomique allant jusqu'à 35 inclus, R₃ représente l'hydrogène ou un groupe alkyle en C1-C7, le radical benzohétérocyclyle représenté par la formule Ia est un radical 2,3-dihydrobenzofurannyle, 2,3-dihydrobenzothiényle, chromannyle, thiochromannyle, 1,4-benzodioxannyle, 1,4-benzoxathiannyle, 1,4-benzodithiannyle, 2,3,4,5-tétrahydrobenzoxépinyle, 2,3,4,5-tétrahydrobenzothiépinyle, 2,3,4,5-tétrahydrobenzodioxépinyle, 2,3,4,5-tétrahydrobenzoxathiépinyle ou 2,3,4,5-tétrahydrobenzodithiépinyle, et alk₁ et alk₂, ayant des significations identiques ou différentes, représentent chacun un groupe alkylène en C1-C4, et leurs sels.

5. Composés selon revendication 2, répondant à la formule I dans laquelle R₁ et R₂ représentent chacun, indépendamment l'un de l'autre, l'hydrogène, un groupe alkyle en C1-C4, alcoxy en C1-C4 ou un halogène de numéro atomique allant jusqu'à 35 inclus, R₃ représente l'hydrogène ou un groupe alkyle en C1-C4, le radical benzohétérocyclyle représenté par la formule Ia est un radical 2,3-dihydrobenzofurannyle, chromannyle ou 1,4-benzodioxannyle, alk₁ représente un groupe alkylène en C1-C4 et alk₂ un groupe alkylène en C2-C3, et leurs sels.

6. Composés selon revendication 2, répondant à la formule I dans laquelle R₁ représente l'hydrogène, un groupe alkyle en C1-C4, alcoxy en C1-C4 ou un halogène de numéro atomique allant jusqu'à 35 inclus, R₂ représente l'hydrogène, R₃ l'hydrogène ou un groupe alkyle en C1-C4, le radical benzohétérocyclyle représenté par la formule Ia est un radical 2,3-dihydrobenzofurannyle, chromannyle ou 1,4-benzodioxannyle, alk₁ représente un groupe alkylène en C1-C3 et alk₂ un groupe éthylène, et leurs sels.

7. Composé selon revendication 1, qui consiste en l'acide 3-{N-[2-(chromanne-3-yl)-éthyl]-amino}-1-hydroxypropane-1,1-diphosphonique ou l'un de ses sels.

8. Composé selon revendication 1, qui consiste en l'acide 3-{N-[2-(chromanne-3-yl)-éthyl]-N-méthyl-amino}-1-propane-1,1-diphosphonique ou l'un de ses sels.

9. Composé selon revendication 1, qui consiste en l'acide 3-[N-(6-méthyl-1,4-benzodioxanne-2-yl)-méthyl-N-méthyl-amino]-1-hydroxy-propane-1,1-diphosphonique ou l'un de ses sels.

10. Composé selon revendication 1, qui consiste en l'acide 3-[N-(6-méthoxy-1,4-benzodioxanne-2-yl)-méthyl-N-méthyl-amino]-1-hydroxy-propane-1,1-diphosphonique ou l'un de ses sels.

11. Composé selon revendication 1, qui consiste en l'acide 3-[N-(7-chloro-1,4-benzodioxanne-2-yl)-méthyl-N-méthyl-amino]-1-hydroxy-propane-1,1-diphosphonique ou l'un de ses sels.

12. Composé selon revendication 1, qui consiste en l'acide 3-[N-(2,3-dihydrobenzothiéne-2-yl)-méthyl-N-méthylamino]-1-hydroxy-propane-1,1-diphosphonique ou l'un de ses sels.

13. Composé selon revendication 1, qui consiste en l'acide 3-{N-[2-(chromanne-2-yl)-éthyl]-N-méthyl-amino}-1-hydroxy-propane-1,1-diphosphonique ou l'un de ses sels.

14. Composé selon revendication 1, qui consiste en l'acide 3-[N-(6-méthylbenzo-1,4-dioxanne-2-ylméthyl)-amino]-1-hydroxy-propane-1,1-diphosphonique ou l'un de ses sels.

15. Composé selon revendication 1, qui consiste en l'acide 3-[N-(benzo-1,4-dioxanne-2-ylméthyl)-N-méthyl-amino]-1-hydroxypropane-1,1-diphosphonique ou l'un de ses sels.

16. Composé selon revendication 1, qui consiste en l'acide 3-[N-(5-méthoxy-2,3-dihydro-benzofuranne-2-yl)méthylamino]-1-hydroxy-propane-1,1-diphosphonique ou l'un de ses sels.

17. Composé selon revendication 1, qui consiste en l'acide 3-[N-(6-chlorobenzo-1,4-dioxanne-2-ylméthyl)-N-méthyl-amino]-1-hydroxy-propane-1,1-diphosphonique ou l'un de ses sels.

18. Composés selon l'une des revendications 1 à 17 pour l'utilisation dans un procédé pour le traitement thérapeutique de l'organisme humain ou animal.

19. Compositions pharmaceutiques contenant, avec des produits auxiliaires pharmaceutiques, un composé selon l'une des revendications 1 à 18 à l'état libre ou à l'état de sel acceptable pour l'usage pharmaceutique, en tant que substance active pharmaceutique.

20. Procédé de préparation des acides benzohétérocyclylalkylaminoalcane-diphosphoniques selon revendication 1 ou de leurs sels, caractérisé en ce que
a) dans un composé de formule II 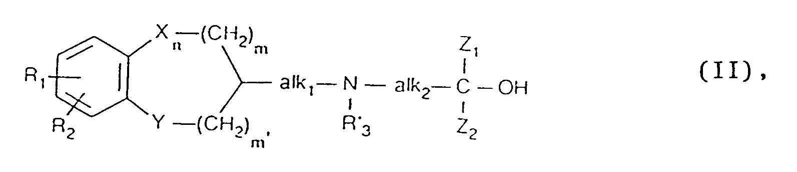 dans laquelle R₁, R₂, n, m, m', X, Y, alk₁ et alk₂ ont les significations indiquées ci-dessus, R'₃ représente l'un des groupes mentionnés en référence à R₃ ou un groupe protecteur du groupe amino R₀, Z représente un groupe phosphono ayant subi une modification fonctionnelle et Z₂ un groupe phosphono libre ou ayant subi une modification fonctionnelle, on convertit le groupe phosphono Z₁, ayant subi une modification fonctionnelle, et le cas échéant, le groupe Z₂, ayant subi une modification fonctionnelle, en groupes phosphono libres, ou bien
b) on fait réagir entre eux des composés de formules IIIa et IVa 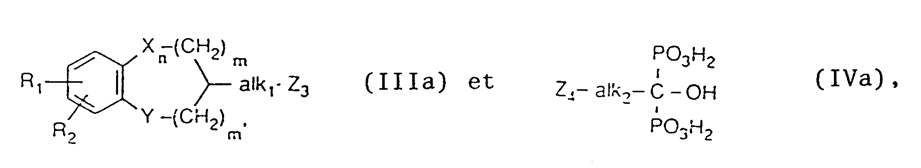 dans lesquelles R₁, R₂, n, m, m', X, Y, alk₁ et alk₂ ont les significations indiquées ci-dessus, l'un des symboles Z₃ et Z₄ représente un groupe hydroxy estérifié réactif et l'autre un groupe de formule -N(R'₃)-H dans lequel R'₃ représente l'un des groupes mentionnés en référence à R₃ ou un groupe protecteur du groupe amino R₀, ou leurs sels, ou bien, dans des conditions réductrices, des composés de formules IIIb et IVb 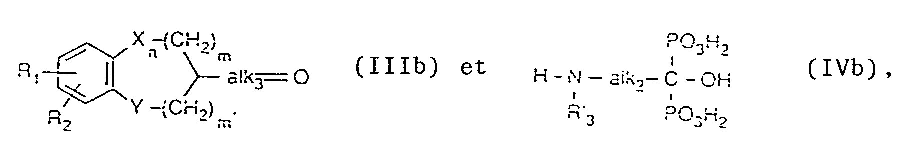 dans lesquelles alk₃ représente un groupe correspondant au groupe alk₁ mais à double liaison, c'est-à-dire un groupe alcanylylidène en C1-C7, R'₃ représente l'un des groupes mentionnés en référence à R₃ ou un groupe protecteur du groupe amino R₀ et R₁, R₂, n, m, m', X, Y, alk₁ et alk₂ ont les significations indiquées ci-dessus, ou bien
c) on fait réagir un composé de formule V 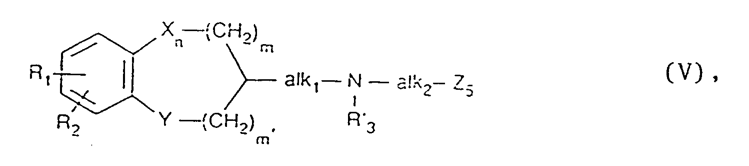 dans laquelle Z₅ représente un groupe carboxy, carbamoyle ou cyano et R'₃ l'un des groupes mentionnés en référence à R₃ ou un groupe protecteur du groupe amino R₀, et R₁, R₂, n, m, m', X, Y, alk₁ et alk₂ ont les significations indiquées ci-dessus, avec un agent phosphorylant, on hydrolyse le produit obtenu en premier et, dans un produit intermédiaire de formule Va, 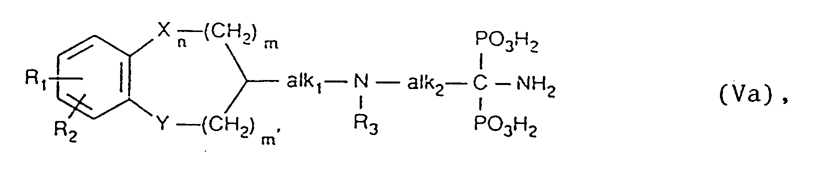 ou de l'un de ses sels, obtenu au départ de composés de formule V dans laquelle Z₅ représente un groupe cyano ou carbamoyle, on remplace le groupe amino par un groupe hydroxy en traitant par l'acide nitreux, on élimine le groupe protecteur éventuel du groupe amino R₀ et si on le désire, on convertit un composé ainsi obtenu en un autre composé de formule I, on sépare un mélange d'isomères ainsi obtenu en les composants et on isole l'isomère préféré dans chaque cas et/ou on convertit un composé libre obtenu comme décrit ci-dessus en un sel ou un sel obtenu comme décrit ci-dessus en le composé libre correspondant.

21. Utilisation d'un composé selon l'une des revendications 1 à 18 pour la préparation d'un médicament prévu pour le traitement de maladies en rapport avec des perturbations du métabolisme du calcium, de processus inflammatoires dans les articulations, de processus dégénératifs des cartilages articulaires, ou encore des dépôts de calcium dans les vaisseaux sanguins ou dans les implants prothétiques, et en particulier des hypercalcémies induites par des tumeurs, des métastases des os et de la maladie de Paget, de l'ostéoporose, de la periodontite et de l'hyperparathyroïdisme.

## Revendications (Revendications pour l'(les) Etat(s) contractant(s) suivant(s): ES, GR)

1. Procédé de préparation des acides benzohétérocyclylalkylaminoalcane-diphosphoniques de formule I 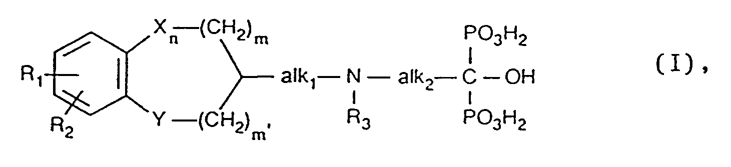 dans laquelle R₁ et R₂ représentent chacun, indépendamment l'un de l'autre, l'hydrogène, un groupe alkyle en C1-C7, alcoxy en C1-C7, un halogène ou un groupe trifluorométhyle, R₃ représente l'hydrogène ou un groupe alkyle en C1-C7, X et Y représentent chacun, indépendamment l'un de l'autre, un pont oxy ou thio, alk₁ et alk₂, ayant des significations identiques ou différentes, représentent chacun un groupe alkylène en C1-C7, n est égal à 0 ou 1 et m et m' sont égaux chacun, indépendamment l'un de l'autre, à 0, 1 ou 2, la somme de n, m et m' étant égale à 1, 2 ou 3, et leurs sels, caractérisé en ce que
a) dans un composé de formule II 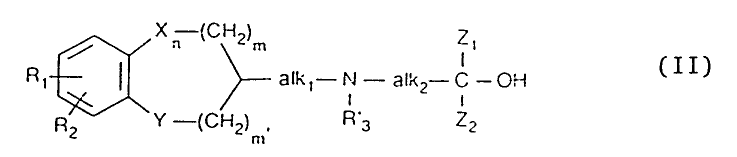 dans laquelle R₁, R₂, n, m, m', X, Y, alk₁ et alk₂ ont les significations indiquées ci-dessus, R'₃ représente l'un des groupes mentionnés en référence à R₃ ou un groupe protecteur du groupe amino R₀, Z représente un groupe phosphono ayant subi une modification fonctionnelle et Z₂ un groupe phosphono libre ou ayant subi une modification fonctionnelle, on convertit le groupe phosphono Z₁, ayant subi une modification fonctionnelle, et le cas échéant, le groupe Z₂, ayant subi une modification fonctionnelle, en groupes phosphono libres, ou bien
b) on fait réagir entre eux des composés de formules IIIa et IVa 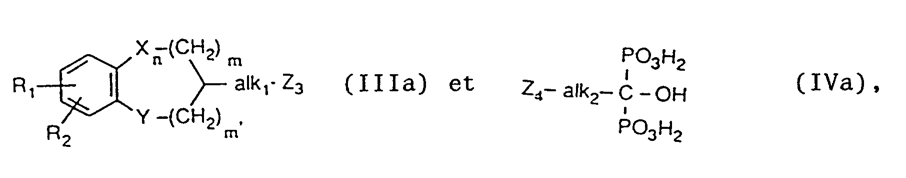 dans lesquelles R₁, R₂, n, m, m', X, Y, alk₁ et alk₂ ont les significations indiquées ci-dessus, l'un des symboles Z₃ et Z₄ représente un groupe hydroxy estérifié réactif et l'autre un groupe de formule -N(R'₃)-H dans lequel R'₃ représente l'un des groupes mentionnés en référence à R₃ ou un groupe protecteur du groupe amino R₀, ou leurs sels, ou bien, dans des conditions réductrices, des composés de formules IIIb et IVb 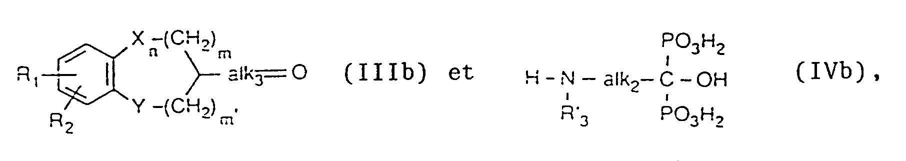 dans lesquelles alk₃ représente un groupe correspondant au groupe alk₁ mais à double liaison, c'est-à-dire un groupe alcanylylidène en C1-C7, R'₃ représente l'un des groupes mentionnés en référence à R₃ ou un groupe protecteur du groupe amino R₀ et R₁, R₂, n, m, m', X, Y, alk₁ et alk₂ ont les significations indiquées ci-dessus, ou bien
c) on fait réagir un composé de formule V 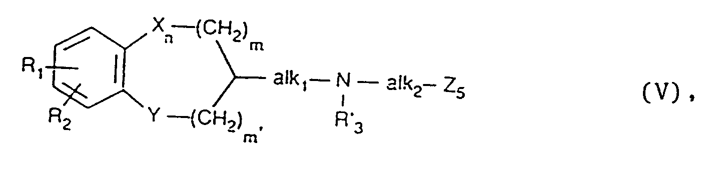 dans laquelle Z₅ représente un groupe carboxy, carbamoyle ou cyano et R'₃ l'un des groupes mentionnés en référence à R₃ ou un groupe protecteur du groupe amino R₀, et R₁, R₂, n, m, m', X, Y, alk₁ et alk₂ ont les significations indiquées ci-dessus, avec un agent phosphorylant, on hydrolyse le produit obtenu en premier et, dans un produit intermédiaire de formule Va, 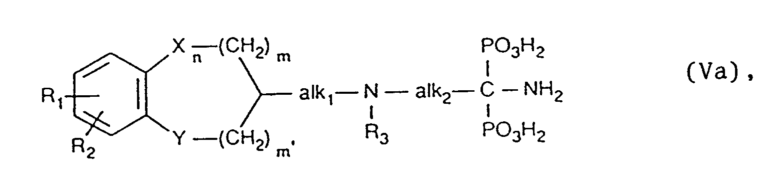 ou de l'un de ses sels, obtenu au départ de composés de formule V dans laquelle Z₅ représente un groupe cyano ou carbamoyle, on remplace le groupe amino par un groupe hydroxy en traitant par l'acide nitreux, on élimine le groupe protecteur éventuel du groupe amino R₀ et si on le désire, on convertit un composé ainsi obtenu en un autre composé de formule I, on sépare un mélange d'isomères ainsi obtenu en les composants et on isole l'isomère préféré dans chaque cas et/ou on convertit un composé libre obtenu comme décrit ci-dessus en un sel ou un sel obtenu comme décrit ci-dessus en le composé libre correspondant.

2. Procédé selon la revendication 1, caractérisé en ce que l'on prépare un composé de formule I dans laquelle R₁ et R₂ représentent chacun, indépendamment l'un de l'autre, l'hydrogène, un groupe alkyle en C1-C7, alcoxy en C1-C7, un halogène ou un groupe trifluorométhyle, R₃ représente l'hydrogène ou un groupe alkyle en C1-C7, le radical benzohétérocyclyle représenté par la formule 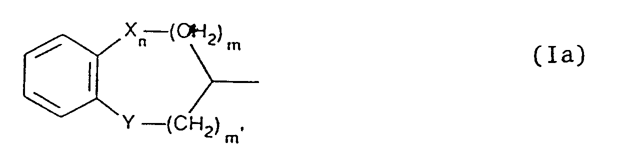 est un radical 2,3-dihydrobenzofurannyle, 2,3-dihydrobenzothiényle, chromannyle, thiochromannyle, 1,4-benzodioxannyle, 1,4-benzoxathiannyle, 1,4-benzodithiannyle, 2,3,4,5-tétrahydrobenzoxépinyle, 2,3,4,5-tétrahydrobenzothiépinyle, 2,3,4,5-tétrahydrobenzodioxépinyle, 2,3,4,5-tétrahydrobenzoxathiépinyle ou 2,3,4,5-tétrahydrobenzodithiépinyle, et alk₁ et alk₂, ayant des significations identiques ou différentes, représentent un groupe alkylène en C1-C7, et leurs sels, les radicaux benzohétérocyclyle mentionnés étant non substitués ou portant un substituant R₁ autre que l'hydrogène ou deux substituants R₁ et R₂ identiques ou différents.

3. Procédé selon revendication 1, caractérisé en ce que l'on prépare un composé de formule I dans laquelle R₁ et R₂ représentent chacun, indépendamment l'un de l'autre, l'hydrogène, un groupe alkyle en C1-C7, alcoxy en C1-C7, un halogène ou un groupe trifluorométhyle, R₃ représente l'hydrogène ou un groupe alkyle en C1-C7, le radical benzohétérocyclyle représenté par la formule 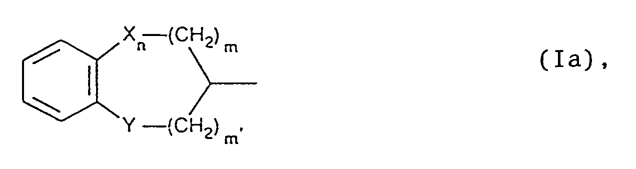 est un radical 2,3-dihydrobenzofurannyle, chromannyle ou 1,4-benzodioxannyle, et alk₁ et alk₂ ayant des significations identiques ou différentes, représentent chacun un groupe alkylène en C1-C7, et leurs sels.

4. Procédé selon revendication 2, caractérisé en ce que l'on prépare un composé de formule I dans laquelle R₁ et R₂ représentent chacun, indépendamment l'un de l'autre, l'hydrogène, un groupe alkyle en C1-C4, alcoxy en C1-C4 ou un halogène de numéro atomique allant jusqu'à 35 inclus, R₃ représente l'hydrogène ou un groupe alkyle en C1-C7, le radical benzohétérocyclyle représenté par la formule Ia est un radical 2,3-dihydrobenzofurannyle, 2,3-dihydrobenzothiényle, chromannyle, thiochromannyle, 1,4-benzodioxannyle, 1,4-benzoxathiannyle, 1,4-benzodithiannyle, 2,3,4,5-tétrahydrobenzoxépinyle, 2,3,4,5-tétrahydrobenzothiépinyle, 2,3,4,5-tétrahydrobenzodioxépinyle, 2,3,4,5-tétrahydrobenzoxathiépinyle ou 2,3,4,5-tétrahydrobenzodithiépinyle, et alk₁ et alk₂, ayant des significations identiques ou différentes, représentent chacun un groupe alkylène en C1-C4, et leurs sels.

5. Procédé selon revendication 2, caractérisé en ce que l'on prépare un composé de formule I dans laquelle R₁ et R₂ représentent chacun, indépendamment l'un de l'autre, l'hydrogène, un groupe alkyle en C1-C4, alcoxy en C1-C4 ou un halogène de numéro atomique allant jusqu'à 35 inclus, R₃ représente l'hydrogène ou un groupe alkyle en C1-C4, le radical benzohétérocyclyle représenté par la formule Ia est un radical 2,3-dihydrobenzofurannyle, chromannyle ou 1,4-benzodioxannyle, alk₁ représente un groupe alkylène en C1-C4 et alk₂ un groupe alkylène en C2-C3, et leurs sels.

6. Procédé selon la revendication 2, caractérisé en ce que l'on prépare un composé de formule I dans laquelle R₁ représente l'hydrogène, un groupe alkyle en C1-C4, alcoxy en C1-C4 ou un halogène de numéro atomique allant jusqu'à 35 inclus, R₂ représente l'hydrogène, R₃ représente l'hydrogène ou un groupe alkyle en C1-C4, le radical benzohétérocyclyle représenté par la formule Ia est un radical 2,3-dihydrobenzofurannyle, chromannyle ou 1,4-benzodioxannyle, alk₁ est un groupe alkylène en C1-C3 tel que méthylène, éthylène ou 1,3-propylène, et alk₂ est un groupe éthylène, ou l'un de ses sels.

7. Procédé selon revendication 2, caractérisé en ce que l'on prépare l'acide 3-{N-[2-(chromanne-3-yl)éthyl]-amino}-1-hydroxy-propane-1,1-diphosphonique ou l'un de ses sels.

8. Procédé selon revendication 2, caractérisé en ce que l'on prépare l'acide 3-{N-[2-(chromanne-3-yl)éthyl]-N-méthyl-amino}-1-propane-1,1-diphosphonique ou l'un de ses sels.

9. Procédé selon revendication 2, caractérisé en ce que l'on prépare l'acide 3-[N-(6-méthyl-1,4-benzodioxanne-2-yl)-méthyl-N-méthyl-amino]-1-hydroxypropane-1,1-diphosphonique ou l'un de ses sels.

10. Procédé selon revendication 2, caractérisé en ce que l'on prépare l'acide 3-[N-(6-méthoxy-1,4-benzodioxanne-2-yl)-méthyl-N-méthyl-amino]-1-hydroxypropane-1,1-diphosphonique ou l'un de ses sels.

11. Procédé selon revendication 2, caractérisé en ce que l'on prépare l'acide 3-[N-(7-chloro-1,4-benzodioxanne-2-yl)-méthyl-N-méthyl-amino]-1-hydroxypropane-1,1-diphosphonique ou l'un de ses sels.

12. Procédé selon revendication 1, caractérisé en ce que l'on prépare l'acide 3-[N-(2,3-dihydrobenzothiéne-2-yl)-méthyl-N-méthyl-amino]-1-hydroxy-propane-1,1-diphosphonique ou l'un de ses sels.

13. Procédé selon revendication 1, caractérisé en ce que l'on prépare l'acide 3-{N-[2-(chromanne-2-yl)éthyl]-N-méthyl-amino}-1-hydroxy-propane-1,1-diphosphonique ou l'un de ses sels.

14. Procédé selon revendication 1, caractérisé en ce que l'on prépare l'acide 3-[N-(6-méthylbenzo-1,4-dioxanne-2-ylméthyl)-amino]-1-hydroxy-propane-1,1-diphosphonique ou l'un de ses sels.

15. Procédé selon revendication 1, caractérisé en ce que l'on prépare l'acide 3-[N-(benzo-1,4-dioxanne-2-ylméthyl)-N-méthyl-amino]-1-hydroxy-propane-1,1-diphosphonique ou l'un de ses sels.

16. Procédé selon revendication 1, caractérisé en ce que l'on prépare l'acide 3-[N-(5-méthoxy-2,3-dihydrobenzofuranne-2-yl)-méthylamino]-1-hydroxy-propane-1,1- diphosphonique ou l'un de ses sels.

17. Procédé selon revendication 1, caractérisé en ce que l'on prépare l'acide 3-[N-(6-chlorobenzo-1,4-dioxanne-2-ylméthyl)-N-méthyl-amino]-1-hydroxy-propane-1,1-diphosphonique ou l'un de ses sels.

18. Procédé de préparation de compositions pharmaceutiques appropriées au traitement des perturbations du métabolisme du calcium, caractérisé en ce que l'on met un composé obtenu selon l'une des revendications 1 à 17, de manière connue en soi, seul ou avec des produits auxiliaires pharmaceutiques usuels pour la préparation de médicaments, éventuellement en combinaison avec d'autres substances actives médicamenteuses, sous la forme de comprimés, de capsules, de pilules, de suppositoires, d'émulsions, de solutions pour injections ou perfusions, ou sous d'autres formes médicamenteuses connues.
